# EUROPEAN PATENT APPLICATION

(11) **EP 4 438 600 A1**
(43) Date of publication of application: **02.10.2024**
(21) Application number: 23777843.6
(22) Date of filing: 15.03.2023
(51) Int. Cl.: C07D 403/14, C07D 413/14, C07D 401/14, A61P 35/00

(54) **COMPOUND USED AS KINASE INHIBITOR AND USE THEREOF**

(30) Priority: 01.04.2022 CN 202210348367
(71) Applicant: TYK Medicines Inc., Huzhou, Zhejiang 313100 (CN); TYK Medicines (Zhengzhou), Inc., Zhengzhou, Henan 451162 (CN)
(72) Inventor: LI, Jun, Huzhou, Zhejiang 313100 (CN); WU, Yusheng, Huzhou, Zhejiang 313100 (CN); NIU, Chengshan, Zhengzhou, Henan 451162 (CN); GUO, Zhongwei, Zhengzhou, Henan 451162 (CN); LIANG, Apeng, Huzhou, Zhejiang 313100 (CN)
(74) Representative: dompatent von Kreisler Selting Werner - Partnerschaft von Patent- und Rechtsanwälten mbB
(86) International application number: PCT/CN2023/081557
(87) International publication number: WO 2023/185468

(57) **Abstract**

The present invention belongs to the field of synthesis of heterocyclic compound drugs with a nitrogen atom as a heterocyclic atom; particularly relates to a compound used as a kinase inhibitor and the use thereof; and also relates to the preparation of a free base crystal form of the compound. The compound used as a kinase inhibitor is a compound as shown in formula I, or a deuterated compound thereof, or a pharmaceutically acceptable salt, solvate or prodrug. Biological activity experiments prove that the above compound has a good inhibitory activity on exon 20 insertion mutations in EGFR and HER2, exon 19 deletion in EGFR and exon 21 point mutation in EGFR, and can be used as the bulk drug in relevant drugs.

## Description

### Technical field

The present invention belongs to the field of pharmaceutical synthesis of heterocyclic compound with nitrogen atom as heterocyclic atom, and specifically relates to a class of compounds used as kinase inhibitor and use thereof.

### Background

Epidermal growth factor receptor (EGFR) belongs to the receptor tyrosine kinase (RTK) family, which includes EGFR/ERBB1, HER2/ERBB2/NEU, HER3/ERBB3, and HER4/ERBB4. EGFR activates its tyrosine kinase activity through homodimerization or heterodimerization, followed by the phosphorylation of its substrate, thus activates several related downstream pathways in the cell, such as the PI3K-AKT-mTOR pathway involved in cell survival and the RAS-RAF-MEK-ERK pathway involved in cell proliferation. Mutations or amplification of the epidermal growth factor receptor can lead to activation of the epidermal growth factor receptor kinase, which in turn leads to the development of a variety of human diseases, such as malignant tumors. For example, in non-small cell lung cancer patients, more than 10% of the American patients have EGFR mutations, while the percentage of Asian patients with EGFR mutations can reach nearly 50%. Meanwhile, the prevalence of HER2 mutations in non-small cell lung cancer patients is about 2-4%.

EGFR mutations mainly include deletions, insertions and point mutations, wherein exon 19 deletion and exon 21 L858R point mutation account for nearly 90% of EGFR mutations. For tumor patients with these EGFR mutations, the currently marketed EGFR-TKIs include the first-generation Iressa, Tarceva, and Icotinib, the second-generation Afatinib and Dacomitinib, and the third-generation Osimertinib. The other 10% of EGFR mutations primarily involve exons 18 and 20 of the EGFR, and exon 20 insertion mutation in EGFR account for about 9% of the overall EGFR mutations. The most common HER2 mutation in tumor patients with HER2 mutations is an exon 20 insertion mutation in HER2.

TAK-788 is therapeutically effective for exon 20 insertion mutations in EGFR and HER2, and the compound is marketed in the U.S., with a 43% objective remission rate from its reported clinical trial results. DZD9008 has recently been reported to be effective for use in the treatment of advanced non-small cell lung cancer with EGFR or HER2 mutations, with an objective remission rate of 40% for EGFR exon 20ins, which is not satisfactory.

### Summary

In order to meet the clinical drug needs of patients with EGFR and HER2 mutations, especially those with EGFR and HER2 exon 20 insertion mutations, WO2021180238 disclosed a series of compounds with excellent activity on exon 20 insertion mutation of EGFR and HER2, exon 19 deletion and exon 21 L858R point mutation in EGFR, which are highly potential to be developed into drugs for the treatment of related diseases, in which involves a class of compounds with a 3-membered-5-membered fused ring amine, which has been further investigated to show that the stereo configuration of the tris-3-membered-5-membered fused ring amine structure is very important, and when converted from trans- to cis-structure, the in vivo efficacy of the compounds will have better potency and good therapeutic effect in different mutations of the related diseases. In subsequent research and development, in order to find more stable free base compounds with higher bioavailability, the present invention has performed polymorphic studies and screening of free base compounds.

A purpose of the present invention is to provide a class of compound used as kinase inhibitors, which belong to the cis-3-membered-5-membered fused ring amine structure and have good inhibitory activity against exon 20 insertion mutations of EGFR and HER2, exon 19 deletion and exon 21 point mutation in EGFR. The compounds of the present invention have better potency and wider applicability than the compounds with the corresponding trans-structure in application WO2021180238.

A second purpose of the present invention is to provide a use of the above compounds in the preparation of medications for the treatment of related diseases caused by EGFR mutations and/or HER2 mutations.

The present invention also relates to the preparation of free base crystalline forms of the above compounds, wherein polymorphic studies were performed so as to identify the practical crystalline forms with high stability and better bioavailability.

In order to achieve the above purposes, the technical solutions adopted in the present invention are:
A class of compound for use as kinase inhibitor, the compound for use as kinase inhibitor is a compound of formula 1, or a deuterated compound thereof, or a pharmaceutically acceptable salt, a solvate or a prodrug thereof: 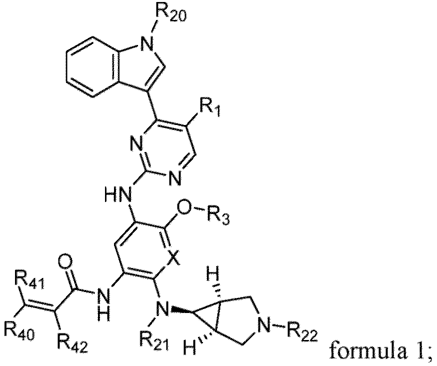 in formula 1, X is selected from CH and N;
R₁ is selected from 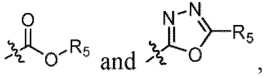 and R₅ is H, C1-C3alkyl, C1-C3fluoroalkyl;
R₂₀, R₂₁, and R₂₂ are each independently selected from methyl and deuteromethyl;
R₃ is selected from C1-C3 alkyl and C1-C3 haloalkyl;
R₄₀, R₄₁, and R₄₂ are each independently selected from H, D and F.

The above compound has good inhibitory activity against EGFR and HER2 mutations as confirmed by bioactivity assays, and can be used as API for related drugs.

On the basis of the above API, a "pharmaceutically acceptable salt" of an API refers to a pharmaceutically acceptable salt that possesses a desired pharmacological activity of the parent compound. Such salts include:
Acid addition salts formed with an inorganic acid, the inorganic salt is, for example, hydrochloric acid, hydrobromic acid, hydriodic acid, sulfuric acid, nitric acid, phosphoric acid and the like; typical inorganic acid salt is selected from hydrochloride, hydrobromide, hydriodate, sulfate, bisulfate, nitrate, phosphate, and acid phosphate. Acid addition salts formed with an organic acid, the organic salt is, for example, formic acid, acetic acid, propionic acid, hexanoic acid, cyclopentane propionic acid, glycolic acid, pyruvic acid, lactic acid, propanedioic acid, succinic acid, malic acid, maleic acid, fumaric acid, tartaric acid, citric acid, benzoic acid, 3-(4-hydroxybenzoyl)benzoic acid, cinnamic acid, mandelic acid, methanesulfonic acid, ethanesulfonic acid, 1,2-ethanedisulfonic acid, 2-hydroxyethanesulfonic acid, benzenesulfonic acid, 4-chlorobenzenesulfonic acid, 2-naphthalenesulfonic acid, 4-methylbenzenesulfonic acid, camphorsulfonic acid, glucoheptonic acid, 4,4'-methylenebis-(3-hydroxy2-en-1-carboxylic acid), 3-phenylpropionic acid, trimethylacetic acid, tert-butylacetic acid, dodecyl sulfuric acid, gluconic acid, glutamic acid, hydroxynaphthoic acid, salicylic acid, stearic acid, muconic acid, etc.; or a salt formed by the coordination of the acidic proton present in the parent compound with an organic base (e.g., ethanolamine, diethanolamine, triethanolamine, aminobutanetriol, N-methylglucosamine, etc.). Typical organic acid salt is selected from formate, acetate, trifluoroacetate, propionate, pyruvate, hydroxyacetate, glycolate, malonate, fumarate, maleate, lactate, malate, citrate, tartrate, methanesulfonate, ethanesulfonate, isethionate, benzenesulfonate, salicylate, picrate, glutamate, ascorbate, camphorate, and camphorsulfonate. It is easy to understand that the pharmaceutically acceptable salt is non-toxic.

The solvate is a compound containing solvent molecule, such as hydrate, dimethyl sulfoxide compound, and the like.

The prodrug refers to a compound that undergoes a chemical transformation by a metabolic or chemical process to produce a compound, salt, or solvate of the present invention in the treatment of related- diseases.

Preferably, the compound for use as kinase inhibitor is a compound of formula 2, or a deuterated compound thereof, or a pharmaceutically acceptable salt, a solvate or a prodrug thereof:

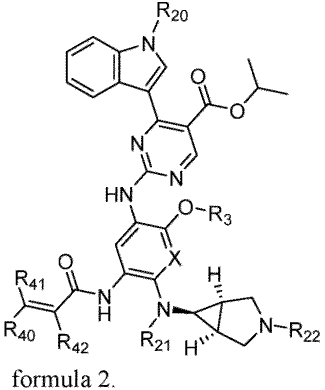

More preferably, in formula 2, X is selected from CH and N; R₃ is selected from -CH₃, - CH₂CH₃ and -CH₂CF₃; R₄₀ and R₄₁ are H, and R₄₂ is selected from H and F.

Preferably, the compound for use as kinase inhibitor is a compound of formula 3, or a deuterated compound thereof, or a pharmaceutically acceptable salt, a solvate or a prodrug thereof:

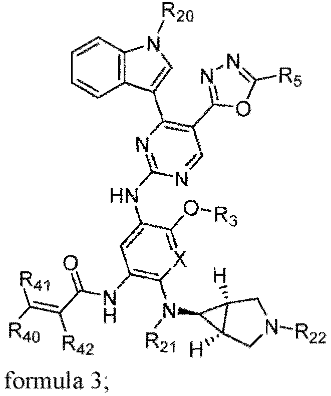

In formula 3, X is selected from CH and N; R₃ is -CH₃, -CH₂CH₃, or -CH₂CF₃; R₄₀ and R₄₁ are H, R₄₂ is selected from Hand F; R₅ is selected from -CH₃ and -CF₃.

Preferably, the compound of formula 1 is: 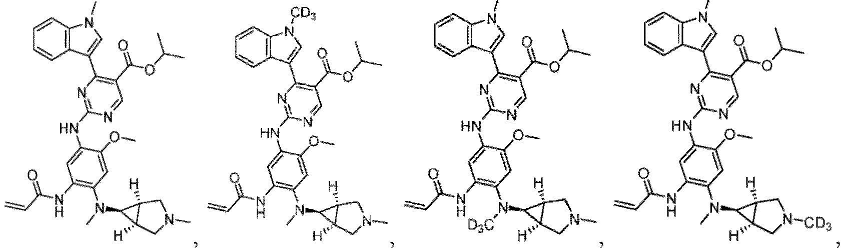 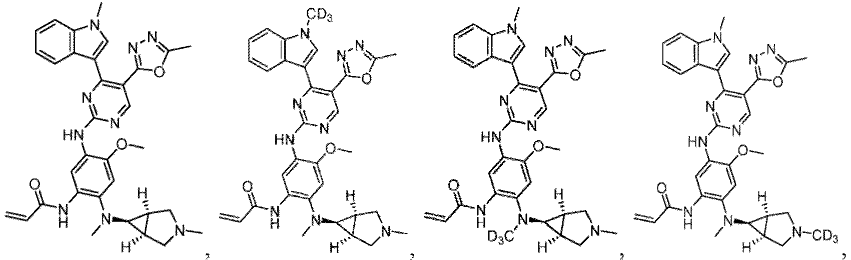 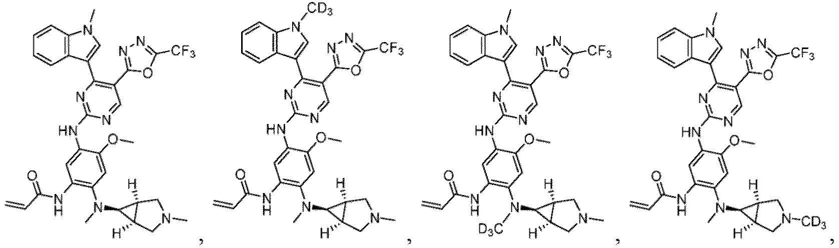 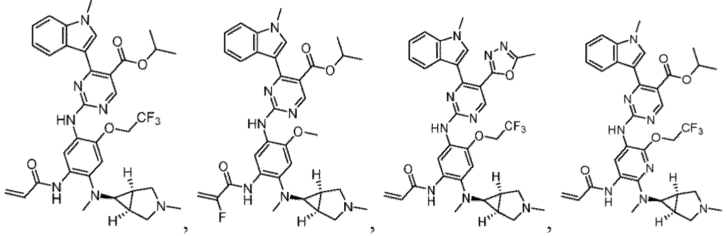 or

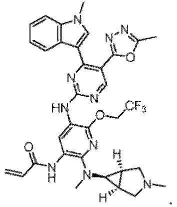

A use of the above compound used as kinase inhibitor in medication for the treatment of related diseases caused by EGFR mutations and/or HER2 mutations.

The compounds described above have good inhibitory activity against exon 20 insertion mutations of EGFR and HER2, and also against exon 19 deletion and exon 21 point mutations in EGFR.

Preferably, the EGFR mutation and/or HER2 mutation comprises one or a combination of two or more of exon 20 insertion mutation in EGFR, exon 20 insertion mutation in HER2, exon 19 deletion in EGFR, exon 20 point mutation in EGFR, and exon 21 point mutation in EGFR. It has been confirmed by biological activity experiments that the above compounds have a better inhibitory effect on the above mutation types.

Further preferably, the EGFR mutation and/or HER2 mutation is selected from EGFR Del 19/T790M/C797S mutation, EGFR L858R/T790M/C797S mutation, and the above compound has better inhibitory effect on the above mutation types.

Preferably, the disease is a cancer caused by the EGFR mutation and/or HER2 mutation. The above compounds can also be used in combination with other drug for the treatment of cancer. The other drug used in combination may be ERK inhibitors or MEK inhibitors.

Preferably, the compound is a crystalline, an amorphous or a solvate; the solvent contained in the solvate is a non-aqueous solvent or a mixture of non-aqueous solvent and water.

In order to better improve the stability and activity of the compound, some of the compound of formula 1 is selected for crystalline studies. Further, a polycrystalline screening study was performed on the compound of Example 1 of the present invention to find a stable and reliable crystalline form, which will both ensure the stability of the quality of the compound and will lead to a better performance of the drug in clinical treatment. The product of Example 1 was found to be a crystal with good crystallinity and an anhydrous crystalline form, named free base crystalline form I. The compound of Example 1 has a compound of Formula A:

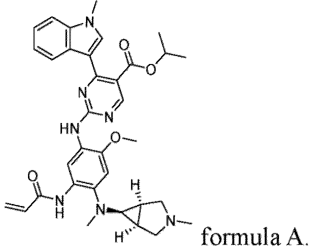

The crystalline form is free base crystalline form I, and the X-ray powder diffraction pattern of the crystalline form has characteristic diffraction peaks at 9.76° ± 0.2°, 10.45° ± 0.2°, 16.54° ± 0.2°, 18.66° ± 0.2°, 20.07° ± 0.2°, and 25.90° ± 0.2°.

Furthermore, the free base crystalline form I, the X-ray powder diffraction pattern of which has characteristic diffraction peaks at 9.07° ± 0.2°, 9.76° ± 0.2°, 10.45° ± 0.2°, 11.53° ± 0.2°, 11.80° ± 0.2°, 12.91° ± 0.2°, 13.79° ± 0.2°, 14.67° ± 0.2°, 15.08° ± 0.2°, 15.63° ± 0.2°, 16.54° ± 0.2°, 17.50° ± 0.2°, 18.66° ± 0.2°, 20.07° ± 0.2°, 21.10° ± 0.2°, 23.29° ± 0.2°, 24.16° ± 0.2°, and 25.90° ± 0.2°.

The free base crystalline form I of the compound of Example 1 can be prepared in most solvents by suspension crystal transformation, anti-solvent precipitation, high and low temperature cycling and evaporating crystallization. Solvents herein include, but are not limited to, one or a mixture of two or more of dichloromethane, 1,4-dioxane, dichloroethane, tert-butyl methyl ether, N-methylpyrrolidone, ethyl acetate, acetone, methanol, dimethyl sulfoxide, isopropyl acetate, butanone, cyclohexane, tetrahydrofuran, water, acetonitrile, isopropanol, ethanol, n-heptane, and the like.

The present invention found that the compound of Example 1 had two anhydrous crystalline forms (free base crystalline form I and free base crystalline form V) and three solvates, of which free base crystalline form II was an ethanol solvate, free base crystalline form III was an isopropanol solvate, and free base crystalline form IV was a solvate of dichloroethane and water, and all of these free base crystalline solvates were characterized. Competition pulping experiment was performed on the two anhydrous crystalline forms, i.e., free base crystalline form I and free base crystalline form V From the results, it was inferred that free base crystalline form I might be a thermodynamically stable crystalline form, which is more suitable for subsequent development.

The crystalline evaluation of free base crystalline form I was performed, including dry grinding, wet grinding, tablet pressing (30 MPa), studies of stability and hygroscopicity, ect.. It became amorphous after 5 min of dry grinding; the crystalline form was basically unchanged after wet grinding with water for 5 min, and the crystallinity slightly decreased after 5 min of wet grinding with ethanol; the crystallinity decreased when the pressure was 30 MPa in the tablet pressing test; in the stability study, the free base crystalline form I was relatively stable at different temperatures and humidity, and there was no change in the results of the liquid chromatography detection; the dynamic water vapor adsorption (DVS) test was performed, and the results showed that at 80 % RH, the weight gain by water absorption was 0.66%, and the free base crystalline form I was slightly hygroscopic. In conclusion, the free base crystalline form I is a relatively stable anhydrous crystalline form with stable solid state properties and slight hygroscopicity, which can be used for subsequent drug development.

### Description of the drawings

Figure 1 shows the tumor volume changes (mm³) in the LU0387 model;
Figure 2 shows the tumor volume change (mm³) in the Ba/F3 EGFR D770_N771 ins SVD model;
Figure 3 shows the single crystal diffraction pattern of the single crystal of Example 1;
Figure 4 shows the XRPD pattern of free base crystalline form I;
Figure 5 shows the DSC and TGA overlay pattern of free base crystalline form I;
Figure 6 shows the XRPD pattern of the amorphous sample obtained by dry grinding method;
Figure 7 shows the XRPD pattern of free base crystalline form II;
Figure 8 shows the DSC and TGA overlay pattern of free base crystalline form II;
Figure 9 shows the XRPD pattern of free base crystalline form III;
Figure 10 shows the DSC and TGA overlay pattern of free base crystalline form III;
Figure 11 shows the XRPD pattern of free base crystalline form IV;
Figure 12 shows the DSC and TGA overlay pattern of free base crystalline form IV;
Figure 13 shows the XRPD overlay pattern of free base crystalline form V;
Figure 14 shows the DSC and TGA overlay pattern of free base crystalline form V

### Detailed Description of the Invention

In comparison to the compounds having trans-3-membered-5-membered fused ring amine structure in application WO2021180238, the present invention confirms that the cis-3-membered-5-membered fused ring amine structure has better in vivo efficacy and is also therapeutically effective in different mutations of the related disease.

The preparation method for the compound of the present invention: when X is CH, the reaction route is shown below: 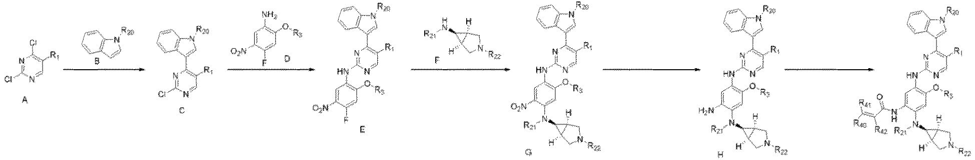 including the following steps:
(1) Compound A and compound B undergo a Friedel-Crafts reaction in an organic solvent to obtain compound C;
(2) Compound C and compound D undergo a substitution reaction in the presence of an acid catalyst in an organic solvent to obtain compound E;
(3) Compound E and compound F undergo a substitution reaction in the presence of a base catalyst in an organic solvent to obtain compound G;
(4) Compound G is reduced to obtain Compound H;
(5) Compound H undergoes a condensation reaction in the presence of a base catalyst to obtain the product.

When R₁ is 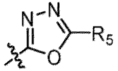 and R₅ is -CF₃, the synthesis of the compound of formula 1 may also follow the above reaction route: 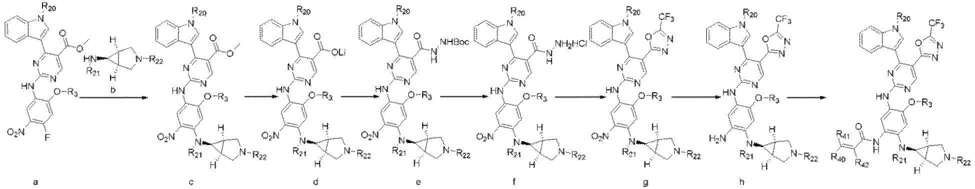 including the following steps:
(1) Compound a and compound b undergo a substitution reaction in an organic solvent to obtain compound c;
(2) Compound c is hydrolyzed to obtain compound d;
(3) Compound d undergoes a condensation reaction to obtain compound e;
(4) Compound e is deprotected to obtain compound f;
(5) Compound f undergoes a ring-closing reaction with trifluoroacetic anhydride in the presence of a base catalyst to obtain compound g;
(6) Compound g is reduced to obtain compound h;
(7) Compound h undergoes a condensation reaction in the presence of a base catalyst to obtain the product.

The preparation method for the compound of the present invention: when X is N, the reaction route is shown below: 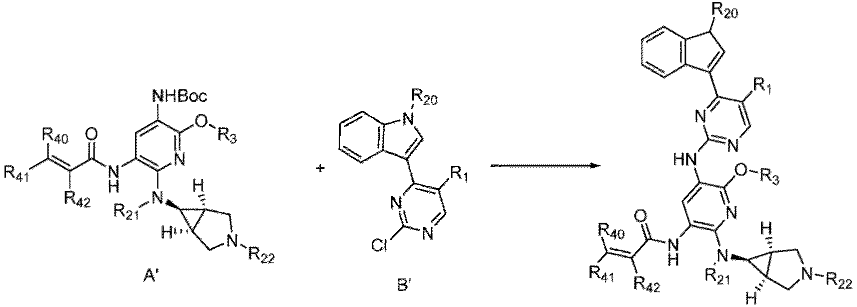 comprising the following steps: compound A' and compound B' undergo a substitution reaction to obtain the product.

Wherein, compound A' is obtained by referring to the synthesis of WO2021180238; compound B' is obtained by Friedel-Crafts reaction.

The processes of the present invention are all described in detail below in conjunction with specific examples.

### I. Specific Examples of the Synthesis of Compounds Used as Kinase Inhibitors

### Example 1

The compound used as kinase inhibitors of this example has the structural formula shown below:

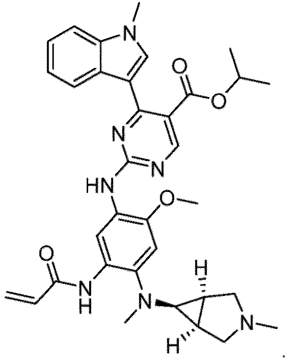

The synthetic route for the compound of this example is as follows:

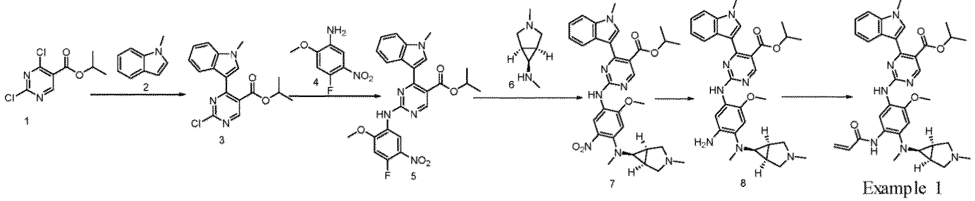

Synthesis of Compound 3: to a 250 mL three-necked flask under nitrogen protection was added compound 1 (2.01g, 8.5mmol), dissolved completely with 100mL of tetrahydrofuran, added with anhydrous aluminum trichloride (2.26g, 17mmol), and stirred at 70°C for 1 hour. Compound 2 (1.34g, 10.2mmol) was added dropwise, then the reaction was continued at 70°C, and monitored by thin-layer chromatography. The reaction was basically completed after 4 hours, and then processed to obtain 1.31g of the product with a yield of 46.4%.

Synthesis of Compound 5: Compound 3 (1.30 g, 3.9 mmol), Compound 4 (0.88 g, 4.68 mmol), and p-toluenesulfonic acid (1.35 g, 7.8 mmol) were added to 65 mL of 1,4-dioxane, then heated up to 80 °C and reacted overnight under N₂ protection. The reaction was monitored by thin-layer chromatography, and after completion, it was processed to obtain 1.50g of product with a yield of 79.7%.

Synthesis of compound 7: Compound 5 (6.0 g, 12.5 mmol), compound 6 (6.3 g, 100 mmol) and N, N-diisopropylethylamine (3.2 g, 50 mmol) were added to 120 mL of N,N-dimethylacetamide, and reacted at 90 °C overnight. The reaction was monitored by thin-layer chromatography, and after the raw material was completely reacted, the resulting solution was processed to obtain 8.0 g of the product. ¹H NMR (400 MHz, Chloroform-*d*) δ 9.53 (s, 1H), 8.97 - 8.75 (m, 1H), 7.96 (s, 1H), 7.84 (s, 1H), 7.79 (d, *J* = 8.0 Hz, 1H), 7.38 (d, *J* = 8.2 Hz, 1H), 7.29 (d, *J* = 7.2 Hz, 1H), 7.20 (t, *J* = 7.6 Hz, 1H), 6.81 (s, 1H), 5.09 (p, *J* = 6.4 Hz, 1H), 4.02 (s, 3H), 3.92 (s, 3H), 3.84 - 3.63 (m, 1H), 3.02 (t, *J* = 6.4 Hz, 1H), 2.84 (s, 3H), 2.70 (s, 3H), 2.43 (s, 2H), 2.32 (s, 1H), 1.27 (d, *J* = 7.2 Hz, 2H), 1.15 (d, *J* = 6.4 Hz, 6H).

Synthesis of Compound 8: Compound 7 (7.7g, 41.6mmol) and palladium carbon (2.4g, wet palladium carbon 55%) were added to a mixed solvent of 100mL methanol and 100mL ethyl acetate, and reacted at room temperature for 4 hours under hydrogen atmosphere. The reaction was monitored by thin-layer chromatography, after the raw material was completely reacted, the resulting solution was processed to obtain 7.0 g of the product.

Synthesis of Example 1: Compound 8 (7.00 g, 12.6 mmol) and triethylamine (3.82 g, 37.8 mmol) were dissolved in dichloromethane and cooled down to 0 °C under nitrogen protection, followed by dropwise addition of a solution of acryloyl chloride (1.72 g, 18.9 mmol) in dichloromethane. The reaction was kept at 0 °C for 1 h and monitored by thin-layer chromatography, and after the raw material was completely reacted, the resulting solution was processed: i.e., aqueous sodium bicarbonate solution and dichloromethane were added, and stirred to partition; the aqueous phase was extracted once more with dichloromethane, and the organic phases were combined, dried, spun-dried, and subjected to column chromatography. The crude product obtained was firstly dissolved with a little dichloromethane, and then a large amount of solid was precipitated by adding petroleum ether dropwise. After filtration, the filter cake was directly added with methanol and pulped, and then dried to obtain 3.5g of the product finally. [M+H]⁺: 610.8; ¹H NMR (400 MHz, Chloroform-*d*) δ 10.38 (s, 1H), 9.60 (s, 1H), 8.87 (s, 1H), 8.61 (s, 1H), 7.89 (s, 1H), 7.58 (s, 1H), 7.32 (d, *J* = 8.2 Hz, 1H), 7.20 (t, *J* = 7.6 Hz, 1H), 7.13 (t*, J*= 7.6 Hz, 1H), 6.80 (s, 1H), 6.44 (m, 2H), 6.32(m,1H), 5.68 (m, 1H), 5.00 (p, *J* = 6.2 Hz, 1H), 3.94 (s, 3H), 3.88 (s, 3H), 3.06 (s, 2H), 2.66 (s, 3H), 2.61 (m, 3H), 2.36 (s, 3H), 1.67 (s, 2H), 1.03 (d, *J* = 6.4 Hz, 6H).

With reference to Example 1, three deuterated compounds of Example 2, 3 and 4 were synthesized as shown in Table 1 below.

**Table 1 Structures and characterization of the compounds of Examples 2-4**

| No. | Structure | Characterization |
|---|---|---|
| Example 2 | 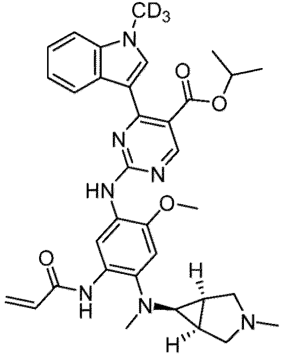 | ¹H NMR (400 MHz, Chloroform-*d*) δ 10.38 (s, 1H), 9.60 (s, 1H), 8.87 (s, 1H), 8.61 (s, 1H), 7.89 (s, 1H), 7.58 (s, 1H), 7.32 (d, *J* = 8.2 Hz, 1H), 7.20 (t, *J* = 7.6 Hz, 1H), 7.13 (t, *J* = 7.6 Hz, 1H), 6.80 (s, 1H), 6.44 (m, 2H), 6.32(m,1H), 5.68 (m, 1H), 5.00 (p, *J* = 6.2 Hz, 1H), 3.88 (s, 3H), 3.06 (s, 2H), 2.66 (s, 3H), 2.61 (m, 3H), 2.36 (s, 3H), 1.67 (s, 2H), 1.03 (d, *J* = 6.4 Hz, 6H). |
| | | [M+H]⁺: 613.8 |
| Example 3 | 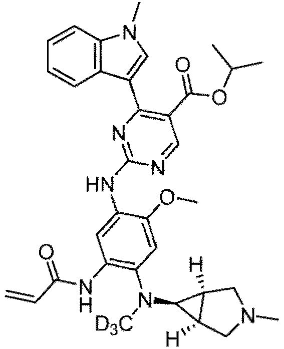 | ¹H NMR (400 MHz, Chloroform-*d*) δ 10.38 (s, 1H), 9.60 (s, 1H), 8.87 (s, 1H), 8.61 (s, 1H), 7.89 (s, 1H), 7.58 (s, 1H), 7.32 (d, *J* = 8.2 Hz, 1H), 7.20 (t, *J* = 7.6 Hz, 1H), 7.13 (t, *J* = 7.6 Hz, 1H), 6.80 (s, 1H), 6.44 (m, 2H), 6.32(m,1H), 5.68 (m, 1H), 5.00 (p, *J* = 6.2 Hz, 1H), 3.94 (s, 3H), 3.88 (s, 3H), 3.06 (s, 2H), 2.61 (m, 3H), 2.36 (s, 3H), 1.67 (s, 2H), 1.03 (d, *J* = 6.4 Hz, 6H). |
| | | [M+H]⁺: 613.8 |
| Example 4 | 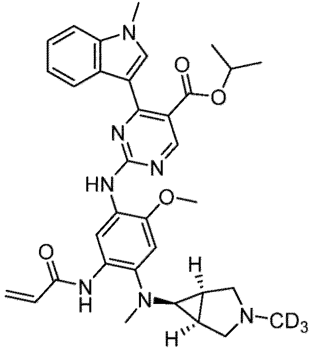 | ¹H NMR (400 MHz, Chloroform-*d*) δ 10.38 (s, 1H), 9.60 (s, 1H), 8.87 (s, 1H), 8.61 (s, 1H), 7.89 (s, 1H), 7.58 (s, 1H), 7.32 (d, *J* = 8.2 Hz, 1H), 7.20 (t, *J* = 7.6 Hz, 1H), 7.13 (t, *J* = 7.6 Hz, 1H), 6.80 (s, 1H), 6.44 (m, 2H), 6.32(m,1H), 5.68 (m, 1H), 5.00 (p, *J* = 6.2 Hz, 1H), 3.94 (s, 3H), 3.88 (s, 3H), 3.06 (s, 2H), 2.66 (s, 3H), 2.61 (m, 3H), 1.67 (s, 2H), 1.03 (d, *J* = 6.4 Hz, 6H). |
| | | [M+H]⁺: 613.8 |

### Example 5

The compound used as kinase inhibitors of this example has the structural formula shown below:

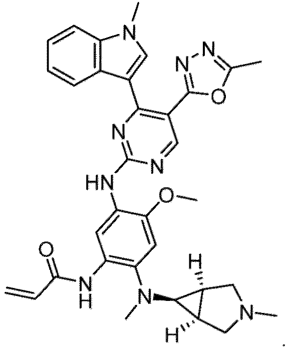

The synthetic route for the compound of this example is as follows:

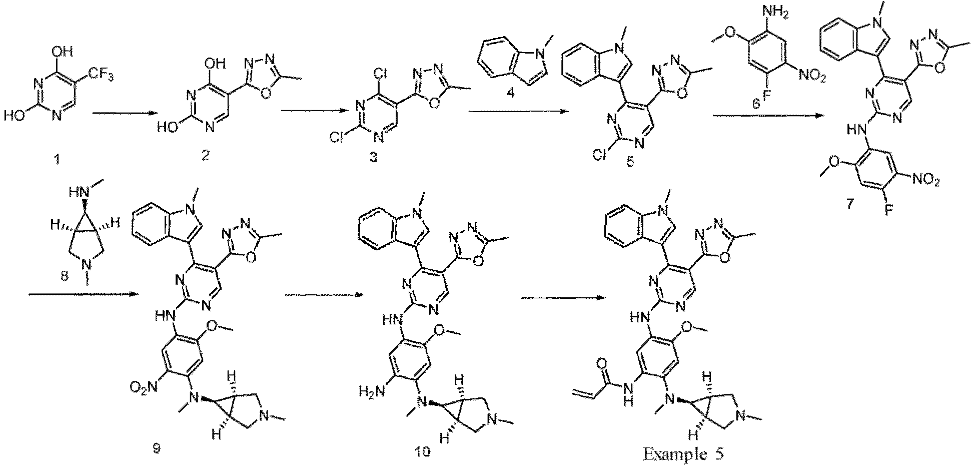

The synthetic route is as follows:
Synthesis of compound 2: to a 2000mL single-necked flask was added compound 1 (54.6g, 303mmol), acetylhydrazine (26.64g, 395mmol), and 1000mL of 1N aqueous sodium hydroxide solution, and then reacted at 80 °C for 4 hours; a large amount of solid precipitated. Then the reaction solution was cooled down, added with 80 mL of concentrated hydrochloric acid, and stirred at low temperature around 0°C for 30 min. After leaching, the filter cake was washed by stirring with water once, and dried in vacuum at 55°C overnight to obtain 32 g of the product. ¹H NMR (400 MHz, DMSO-*d*₆) δ 11.75 (s, 1H), 11.59 (s, 1H), 8.13 (s, 1H), 2.52 (s, 3H).

Synthesis of Compound 3: To a 2000 mL single-necked flask was added compound 2 (50.0 g, 258 mmol), then 1500 mL of toluene, phosphorus oxychloride (237 g, 1550 mmol), and N,N-diisopropylethylamine (133 g, 1031 mmol) were added at room temperature, and white mist was generated. The resulting solution was then heated to 80 °C and stirred overnight under nitrogen protection. On the next day, the reaction solution was cooled down and processed to obtain 30 g of the product. ¹H NMR (400 MHz, Chloroform-*d*) δ 9.19 (s, 1H), 2.71 (s, 3H).

Synthesis of Compound 5: To a 2000 mL single-necked flask was added Compound 3 (30.0 g, 130 mmol), dissolved completely with 1500 mL of 1,2-dichloroethane, and then added with anhydrous Aluminum trichloride (29.3 g,220 mmol). The reaction solution was stirred first at room temperature for 30 minutes and then cooled down to 0 °C. Compound 4 (22.1g,169mmol) was added dropwise and stirred at low temperature for 30 minutes, after which the reaction was warmed up to 60 °C and monitored by thin-layer chromatography. After 4 hours of reaction, the reaction solution was cooled down and processed to give 20.0 g of product. ¹H NMR (400 MHz, Chloroform-*d*) δ 8.84 (s, 1H), 8.02 (dt, *J* = 7.8, 1.0 Hz, 1H), 7.97 (s, 1H), 7.40-7.27 (m, 3H), 3.86 (s, 3H), 2.50 (s, 3H).

Synthesis of Compound 7: Compound 5 (2 g, 6.15 mmol), Compound 6 (1.37 g, 7.38 mmol), and p-toluenesulfonic acid (2.11 g, 12.3 mmol) were added to 100 mL of 1,4-dioxane, then heated up to 80 °C and reacted overnight under N₂ protection. The reaction was monitored by thin-layer chromatography, and after completion, the resulting solution was processed to obtain 0.65 g of product.

Synthesis of compound 9: Compound 7 (950 mg, 2 mmol), compound 8 (1.0 g, 8 mmol) and N,N-diisopropylethylamine (516mg, 4 mmol) were added to 20 mL of N,N-dimethylacetamide, and reacted at 100 °C overnight. The reaction was monitored by thin-layer chromatography, and after the raw material was completely reacted, the resulting solution was processed to obtain 1.0 g of product. ¹H NMR (400 MHz, Chloroform-*d*) δ 9.56 (s, 1H), 8.88 (s, 1H), 7.92 (d, *J* = 14.6Hz, 2H), 7.54 (s, 1H), 7.40 (d, *J* = 8.2 Hz, 1H), 7.32 (s, 1H), 7.15 (t, *J* = 7.6 Hz, 1H), 6.84 (s, 1H), 4.06 (s, 3H), 3.93 (s, 3H), 3.04 (t, *J=* 6.0 Hz, 1H), 2.88 (s, 3H), 2.72 (s, 3H), 2.44 (s, 2H), 2.38 (s, 3H), 1.28 (s, 4H).

Synthesis of Compound 10: Compound 9 (1.0g, 1.72mmol) and palladium carbon (500mg, wet palladium carbon 55%) were added to 50mL methanol, and reacted at room temperature for 4 hours under hydrogen atmosphere. The reaction was monitored by thin-layer chromatography, and after the raw material was completely reacted, the resulting solution was processed to obtain 900 mg of product.

Synthesis of Example 5: Compound 10 (900 mg, 1.63 mmol) and triethylamine (495 mg, 4.90 mmol) were dissolved in dichloromethane and cooled down to 0 °C under nitrogen atmosphere, followed by dropwise addition of a solution of acryloyl chloride (223 mg, 2.45 mmol) in dichloromethane. The reaction was kept at 0 °C for 2 h and monitored by thin-layer chromatography, and after the raw material was completely reacted, the resulting solution was processed to obtain 200 mg of product. [M+H]⁺: 606.8.

With reference to Example 5, three deuterated compounds of Example 6, 7 and 8 were synthesized as shown in Table 2 below.

**Table 2 Structures and characterization of the compounds of Examples 6-8**

| No. | Example 6 | Example 7 | Example 8 |
|---|---|---|---|
| Structure | 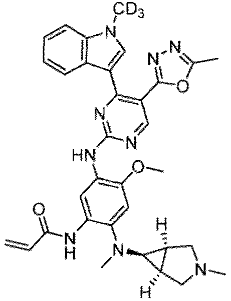 | 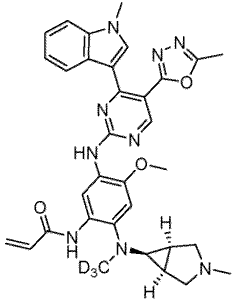 | 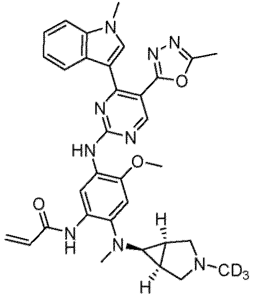 |
| Characterization | [M+H]⁺: 609.8 | [M+H]⁺: 609.8 | [M+H]⁺: 609.8 |

### Example 9

The compound used as kinase inhibitor of this example has the structural formula shown below:

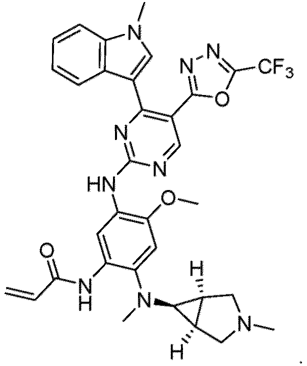

The synthetic route for the compound of this example is as follows:

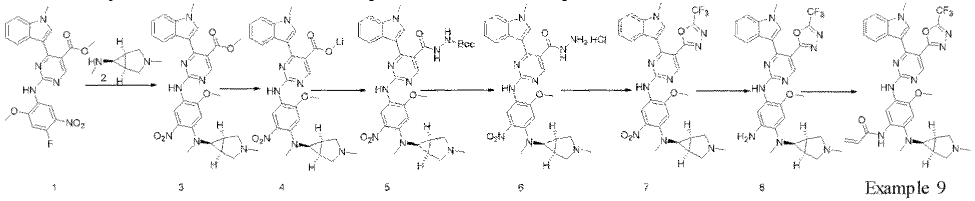

The synthetic route for the compound of this example is as follows:
Synthesis of compound 3: Compound 1 (6.0 g, 13.30 mmol), compound 2 (5.03 g, 39.91 mmol) and N,N-diisopropylethylamine (3.43 g, 26.6 mmol) were added to 60 mL of N,N-dimethylacetamide, and reacted at 80 °C overnight. The reaction was monitored by thin-layer chromatography, and after the raw material was completely reacted, the resulting solution was processed to obtain 6.17g of product. ¹H NMR (400 MHz, Chloroform-*d*)-δ 9.50 (s, 1H), 8.90 (s, 1H), 8.24 -7.99 (m, 1H), 7.80 (s, 1H), 7.67 (s, 1H), 7.38 (dt, *J* = 8.2, 1.0 Hz, 1H), 7.31-7.23 (m, 1H), 7.23-7.16 (m, 1H), 6.65 (s, 1H), 3.95 (s, 3H), 3.94 (s, 3H) , 3.70 (s, 3H), 2.95 (s, 3H), 2.89 (t, *J* = 6.6 Hz, 1H), 2.85-2.76 (m, 2H), 2.27 (m, 2H), 2.15 (s, 3H), 1.91 - 1.81 (m, 2H).

Synthesis of compound 4: Compound 3 (6.17 g, 11.08 mmol), and lithium hydroxide monohydrate (2.33 g, 55.39 mmol) were added to a mixed solvent of tetrahydrofuran/methanol/water in a volume ratio of 6:3:1, and reacted at 40 °C for 16 hours. The reaction was monitored by thin-layer chromatography, and after the raw material was completely reacted, the resulting solution was processed to obtain 6.02 g of product.

Synthesis of compound 5: Compound 4 (6.02 g, 11.08 mmol), tert-butyl hydrazinoformate (4.39 g, 33.26 mmol), N,N-diisopropylethylamine (8.58 g, 66.48 mmol) were added to 160 mL of N,N-dimethylformamide and stirred for 15 min followed by addition of benzotriazole-1-yl-oxytripyrrolidinophosphonium hexafluorophosphate (6.90 g, 13.30 mmol), and then reacted at 25 °C for 16 hours. The reaction was monitored by thin-layer chromatography, and after the raw material was completely reacted, the resulting solution was processed to obtain 1.67 g of product.

Synthesis of compound 6: A mixture of compound 5 (1.67 g, 2.54 mmol) dissolved in 15 mL of tetrahydrofuran was slowly added to 15 mL solution of 4 M HCl in dioxane, and reacted at 40 °C for 5 hours under nitrogen protection. The reaction was monitored by LC-MS, and after the raw material was completely reacted, the resulting solution was processed to obtain 1.20 g of product.

Synthesis of compound 7: Compound 6 (1.0 g, 1.68 mmol), and triethylamine (679 mg, 6.72 mmol) were added to 20 mL of dichloromethane and stirred for 15 min, then trifluoroacetic anhydride (1.59 g, 7.58 mmol) was added in batches and the reaction was reacted for 8 h at 40 °C. After completion of the reaction, the reaction solution was processed to give 521 mg of product.

Synthesis of Compound 8: Compound 7 (521mg, 0.82mmol) and palladium carbon (156mg, 10%) were dissolved in 20mL methanol, and reacted at 25°C for 3 hours under hydrogen atmosphere and stirring. The reaction was monitored by LC-MS, and after the raw material was completely reacted, the resulting solution was processed to obtain 412 g of product.

Synthesis of Example 9: Compound 8 (412 mg, 0.68 mmol) and triethylamine (206 mg, 2.04 mmol) were dissolved in 15ml dichloromethane and cooled down to 0 °C under nitrogen, followed by dropwise addition of a solution of acryloyl chloride (93 mg, 1.02 mmol) in dichloromethane. The reaction was kept at 0 °C for 2 h and monitored by thin-layer chromatography, and after the raw material was completely reacted, the resulting solution was processed to obtain 122 mg of product. [M+H]⁺: 660.5; ¹H NMR (400 MHz, Chloroform-*d*) δ 11.36 (m, 1H), 9.87 (m, 1H), 9.09 (m, 1H), 8.92 (s, 1H), 8.37 (m, 1H), 7.95 (m, 1H), 7.33 (m, 1H), 7.18 (m, 1H), 7.01 (m, 2H), 6.80 (m, 1H), 6.46 (d, J = 16.6 Hz, 1H), 5.74 (d, J = 10.2 Hz, 1H), 3.92 (d, J = 13.5 Hz, 6H), 3.29 (m, 2H), 3.04 (m, 1H), 2.78 (m, 8H), 2.21 (m, 2H).

With reference to Example 9, three deuterated compounds of Example 10, 11 and 12 were synthesized as shown in Table 3 below.

**Table 3 Structures and characterization of the compounds of Examples 10-12**

| No. | Example 10 | Example 11 | Example 12 |
|---|---|---|---|
| Structure | 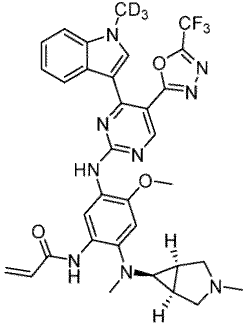 | 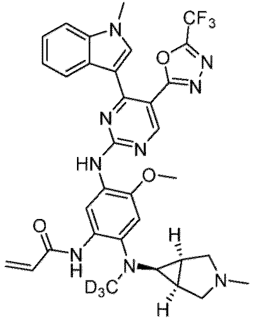 | 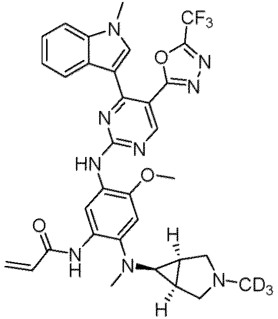 |
| Characterization | [M+H]⁺: 663.5 | [M+H]⁺: 663.5 | [M+H]⁺: 663.5 |

### Example 13

The compound used as kinase inhibitor of this example has the structural formula shown below:

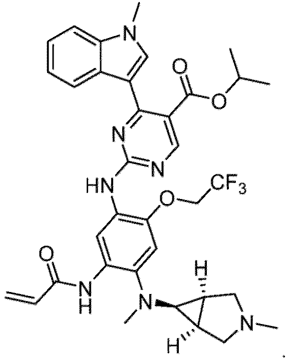

The synthetic route for the compound of this example is as follows:

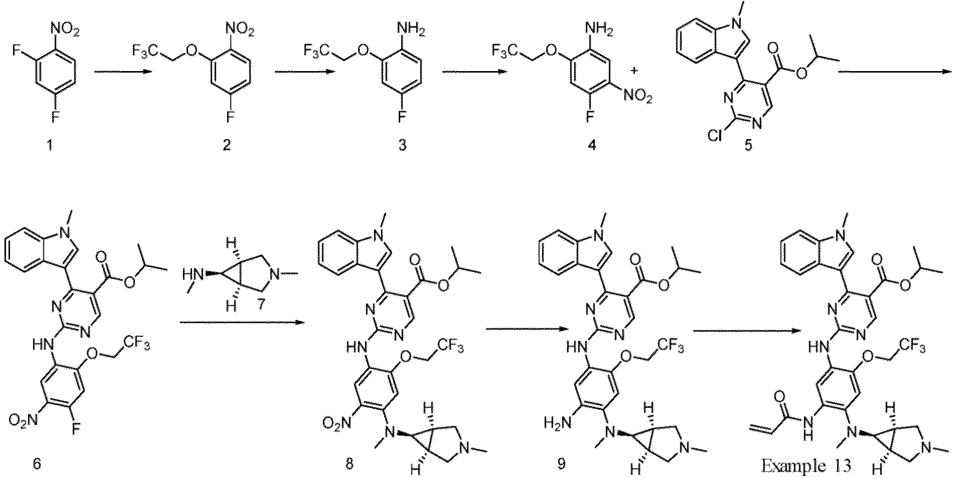

Synthesis of Compound 2: to a 500 mL single-necked flask was added compound 1 (10.00g, 62.88mmol), and dissolved completely with 100mL of tetrahydrofuran; cesium carbonate (20.50g, 62.88mmol), and trifluoroethanol (6.29g, 62.87mmol) were added, and after addition, the resulting solution was protected by nitrogen. The reaction was monitored by thin-layer chromatography, and the reaction was basically completed at 23 °C for 6 hours, and then processed to obtain 14.35g of product with a yield of 95.5%. ¹H NMR (400 MHz, Chloroform-*d*) δ 8.01 (dd, J = 9.1, 5.8 Hz, 1H), 7.09 - 6.72 (m, 2H), 4.50 (q, J = 7.8 Hz, 2H).

Synthesis of Compound 3: Compound 2 (14.00 g, 58.57 mmol) was dissolved in 60 mL of ethanol, and 15 mL of water, ammonium chloride (9.60 g, 179.44 mmol), and reduced iron powder (20.00 g, 357.14 mmol) were added, and then the reaction was heated up to 80 °C and reacted overnight. The reaction was monitored by thin-layer chromatography, and after completion, the resulting solution was processed to obtain 10.50g of product with a yield of 85.78%. ¹H NMR (400 MHz, Chloroform-*d*) δ 6.89 - 6.34 (m, 3H), 4.34 (q, J = 8.0 Hz, 2H), 3.84 - 3.27 (m, 2H).

Synthesis of compound 4: to a 250mL three-necked flask was added compound 3 (10.00g, 47.83mmol), and 50mL of concentrated sulfuric acid was added under stirring. The reaction solution was cooled down to 0°C, and potassium nitrate solid (6.10g, 60.34mmol) was added in batches, after addition, the reaction was protected by nitrogen. After 4 hours of reaction at room temperature, thin-layer chromatography showed that the reaction was completed, and then the resulting solution was processed to obtain 8.26g of product with a yield of 67.98%. ¹H NMR (400 MHz, Chloroform-*d*) δ 7.44 (d, J = 7.2 Hz, 1H), 6.66 (d, J = 11.5 Hz, 1H), 4.46 (q, J = 7.7 Hz, 2H), 4.01 (m, 2H).

Synthesis of Compound 6: Compound 4 (2.00 g, 6.08 mmol) and Compound 5 (2.00 g, 7.87 mmol) were added to 50 mL of acetonitrile, followed by addition of p-toluenesulfonic acid monohydrate (0.81 g, 4.26 mmol), and stirred at 80 °C overnight. On the next day, the reaction was monitored by thin-layer chromatography, and the raw material was basically completely reacted. The reaction solution was cooled down, and solid was precipitated from the reaction solution. After filtration, the filter cake was spin-dried and added into methanol for pulping to obtain 1.67g of product. ¹H NMR (400 MHz, Chloroform-*d*) δ 9.89 (s, 1H), 9.33 (d, J = 8.0 Hz, 1H), 8.77 (s, 1H), 8.29 (s, 1H), 7.67 (d, J = 8.0 Hz, 1H), 7.39 (m, 1H), 7.33 (m, 1H), 7.19 (m, 1H), 6.89 (d, J = 11.3 Hz, 1H), 5.14 (p, J = 6.2 Hz, 1H), 4.59 (q, J = 7.7 Hz, 2H), 3.95 (s, 3H), 1.22 (d, J = 6.2 Hz, 6H).

Synthesis of compound 8: Compound 6 (1.52 g, 2.78 mmol), compound 7 (1.05 g, 8.33 mmol) and N,N-diisopropylethylamine (1.05 g, 8.14 mmol) were added to 25 mL of N,N-dimethylacetamide, and reacted at 90 °C overnight. The reaction was monitored by thin-layer chromatography, and after the raw material was completely reacted, the resulting solution was processed to obtain 1.16 g of product.

Synthesis of Compound 9: Compound 8 (0.59g, 0.90mmol) and palladium carbon (400mg, wet palladium carbon 55%) were added to a mixed solvent of 20mL methanol and 20mL ethyl acetate, and reacted at room temperature for 3 hours under hydrogen atmosphere. The reaction was monitored by thin-layer chromatography, and after the raw material was completely reacted, the resulting solution was processed to obtain 520 mg of product.

Synthesis of Example 13: Compound 9 (520 mg, 0.83 mmol) and triethylamine (253 mg, 2.50 mmol) were dissolved in dichloromethane and cooled down to 0 °C under nitrogen atmosphere, followed by dropwise addition of a solution of acryloyl chloride (114 mg, 1.25 mmol) in dichloromethane. The reaction was kept at 0 °C for 2 h and monitored by thin-layer chromatography, and after the raw material was completely reacted, the resulting solution was processed to obtain 170 mg of product. [M+H]⁺: 606.8. ¹H NMR (400 MHz, Chloroform-*d*) δ 10.63 (s, 1H), 10.11 (s, 1H), 8.82 (s, 1H), 8.16 (s, 1H), 7.66 (m, 2H), 7.29 (d, J = 8.1 Hz, 1H), 7.22 - 7.16 (m, 1H), 7.15 - 7.09 (m, 1H), 6.80 (s, 1H), 6.37 (dd, J = 16.9, 2.0 Hz, 1H), 5.68 (dd, J = 10.1, 2.0 Hz, 1H), 5.00 (p, J = 6.2 Hz, 1H), 4.44 (q, J = 8.2 Hz, 2H), 3.85 (s, 3H), 3.27 - 2.99 (m, 2H), 2.87 - 2.51 (m, 6H), 2.11 (m, 2H), 1.18 - 0.91 (m, 6H).

With reference to Example 13, the compound of Example 14 was synthesized, as shown in Table 4 below.

**Table 4 Structure and characterization of the compound of Example 14**

| No. | Structure | Characterization |
|---|---|---|
| Example 14 | 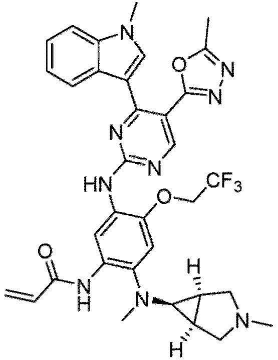 | [M+H]⁺ :674.8 |

### Example 15

The compound used as kinase inhibitor of this example has the structural formula shown below:

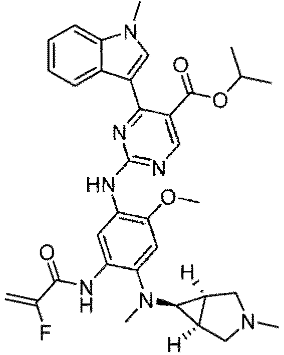

The synthetic route for the compound of this example is as follows:

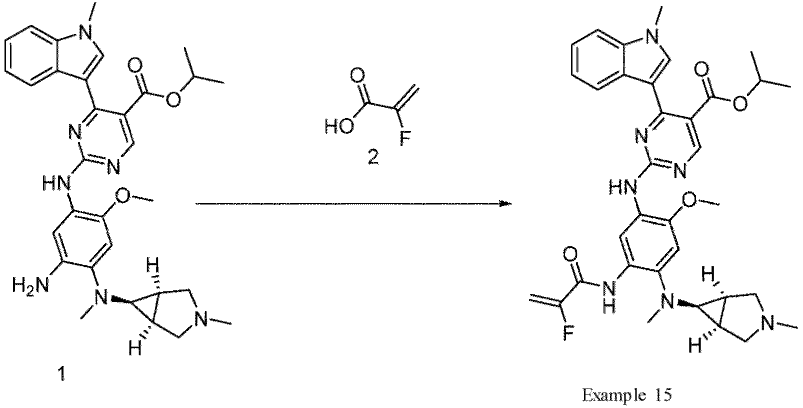

Synthesis of Example 15: To a 50 mL single-necked flask, was added Compound 2 (112 mg, 1.24 mmol), 20 ml of dichloromethane, and oxalyl chloride (142 mg, 1.12 mmol), and stirred for 2 hours under nitrogen protection. The reaction was cooled down to 0 °C, then added dropwise with a solution of compound 1 (438 mg, 78.92 mmol) in dichloromethane and triethylamine (152 mg, 1.50 mmol) and kept at 0 °C for 1 h. The reaction was monitored by thin-layer chromatography, and after the raw material was completely reacted, the resulting solution was processed to obtain 160 mg product finally. [M+H]⁺: 628.8; ¹H NMR (400 MHz, Chloroformed 9.53 (m, 1H), 9.12 (m, 1H), 8.89 (s, 1H), 8.35 (m, 1H), 7.92 (s, 1H), 7.69 (s, 1H), 7.35 (d, J = 8.1 Hz, 1H), 7.23 (t, J = 7.5 Hz, 1H), 7.15 (t, J = 7.5 Hz, 1H), 6.84 (s, 1H), 5.84 (dd, J = 48.2, 3.4 Hz, 1H), 5.28 (dd, J = 15.5, 3.4 Hz, 1H), 5.04 (p, J = 6.2 Hz, 1H), 3.92 (s, 6H), 3.49 (m, 2H), 2.64 (m, 9H), 2.19(m, 2H), 1.09 (m, 6H).

### Example 16

The compound used as kinase inhibitor of this example has the structural formula shown below:

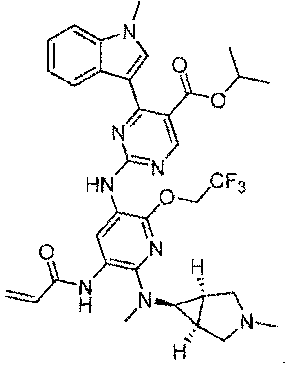

The synthetic route for the compound of this example is as follows:

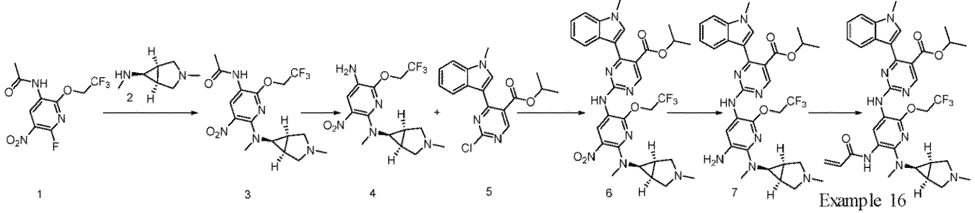

Synthesis of compound 3: to a 100mL three-necked flask was added compound 1 (313mg, 1mmol), 5mL of acetonitrile, and the crude product of compound 2 (441mg), after addition, the mixture was protected by nitrogen. The reaction was monitored by thin-layer chromatography, and was basically completed at 80 °C overnight, then the resulting solution was processed to obtain 300 mg of product.

Synthesis of Compound 4: Compound 3 (1.83 g, 4.54 mmol) was dissolved in 60 mL of methanol, and added with 1.2 mL of concentrated hydrochloric acid. After addition, the reaction was warmed up to 60 °C and reacted overnight. The reaction was monitored by thin-layer chromatography, and after completion, the resulting solution was processed to obtain0.89g of product. ¹H NMR (400 MHz, Chloroform-*d*) δ 7.61 (s, 1H), 4.80 (q, J = 8.4 Hz, 2H), 3.57 (m, 2H), 3.10 (s, 3H), 3.02 (t, J = 6.9 Hz, 1H), 2.59 - 2.33 (m, 4H), 2.06 (s, 3H), 1.76 - 1.51 (m, 2H).

Synthesis of Compound 6: to a 100 mL single-necked flask was added Compound 4 (73.8 mg, 0.21 mmol), Compound 5 (56 mg, 0.17 mmol) and p-toluenesulfonic acid (48 mg, 0.25 mmol), then 5 mL of dioxane was added, and the mixture was stirred at 80 °C overnight under N₂ protection. The reaction was monitored by thin-layer chromatography, and after completion, the resulting solution was processed to obtain 50 mg of product. ¹H NMR (400 MHz, Chloroform-*d*) δ 9.76 (s, 1H), 8.90 (s, 1H), 7.94 (s, 1H), 7.80 (d, J = 8.0 Hz, 1H), 7.56 (s, 1H), 7.39 (d, J = 8.2 Hz, 1H), 7.33 - 7.27 (m, 1H), 7.24 - 7.17 (m, 1H), 5.10 (p, J = 6.2 Hz, 1H), 4.94 (s, 2H), 3.93 (s, 3H), 3.78 (m, 2H), 3.18 (t, J = 6.5 Hz, 1H), 3.01 (m, 3H), 2.57 (m, 5H), 2.31 (m, 2H), 1.16 (d, J = 6.2 Hz, 6H).

Synthesis of Compound 7: Compound 6 (98mg, 0.15mmol) and palladium carbon (80mg, wet palladium carbon 55%) were added to a mixed solvent of 3mL methanol and 2 mL ethyl acetate, and reacted at room temperature for 2 hours under hydrogen atmosphere. The reaction was monitored by thin-layer chromatography, and after the raw material was completely reacted, the resulting solution was processed to obtain 98 mg of product.

Synthesis of Example 16: Compound 8 (300 mg, 0.48 mmol) and triethylamine (150 mg, 1.48 mmol) were dissolved in dichloromethane and cooled down to 0 °C under nitrogen atmosphere, followed by dropwise addition of a solution of acryloyl chloride (70 mg, 0.77 mmol) in dichloromethane. The reaction was kept at 0 °C for 2 h and monitored by thin-layer chromatography, and after the raw material was completely reacted, the resulting solution was processed to obtain 118 mg of product. [M+H]⁺: 688.8. ¹H NMR (400 MHz, Chloroform-*d*) δ 10.39 (s, 1H), 9.97 (s, 1H), 8.89 (s, 1H), 8.59 (m, 1H), 7.59 (m, 2H), 7.33 (m, 1H), 7.23 (m, 1H), 7.14 (m, 1H), 6.45 (m, 1H), 6.31 (m, 1H), 5.72 (d, J = 10.0 Hz, 1H), 5.01 (p, J = 6.5 Hz, 1H), 4.83 (q, J = 8.5 Hz, 2H), 3.96 (s, 3H), 3.14 - 2.88 (m, 2H), 2.85 - 2.52 (m, 6H), 2.33 (s, 3H), 1.69 (m, 2H), 1.04 (d, J = 6.3 Hz, 6H).

### Example 17

The compound used as kinase inhibitor of this example has the structural formula shown below:

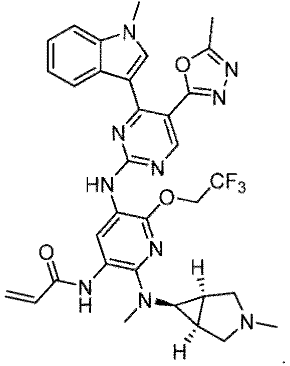

The synthetic route for the compound of this example is as follows:

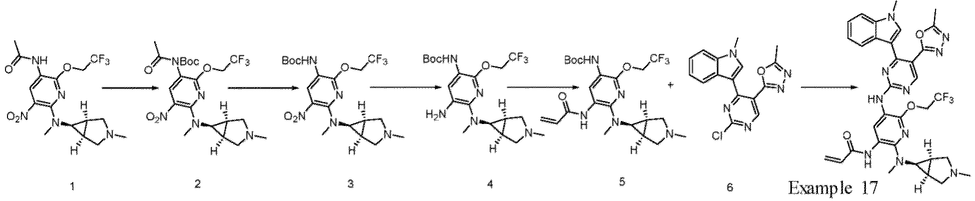

Synthesis of compound 2: to a 100mL three-necked flask was added compound 1 (153mg, 0.38mmol), 5mL of tetrahydrofuran, and 4-dimethylaminopyridine (17.8mg). After addition, the mixture was protected by nitrogen, and di-tert-butyl dicarbonate (1.06g, 4.86mmol) was added dropwise. The reaction was monitored by thin-layer chromatography, and was basically completed at 80 °C for 2h, then the resulting solution was processed to obtain 104 mg of product.

Synthesis of Compound 3: Compound 2 (104 mg, 0.21 mmol) was dissolved in 2 ml of methanol, and 0.05 mL solution of 5.4 mol/L sodium methoxide in methanol was added, after addition, the reaction was carried out for 30 min. The reaction was monitored by thin-layer chromatography, and after completion, the resulting solution was processed to obtain 30mg of product.

Synthesis of Compound 4: Compound 3 (30mg, 0.065mmol) and palladium carbon (15mg, wet palladium carbon 55%) were added to a mixed solvent of 1mL methanol and 1mL ethyl acetate, and reacted at room temperature for 2 hours under hydrogen atmosphere. The reaction was monitored by thin-layer chromatography, and after the raw material was completely reacted, the resulting solution was processed to obtain 30 mg of product.

Synthesis of Example 5: Compound 4 (30 mg, 0.069 mmol) and triethylamine (13 mg, 0.14 mmol) were dissolved in dichloromethane and cooled down to 0 °C under nitrogen atmosphere, followed by dropwise addition of a solution of acryloyl chloride (7 mg, 0.077 mmol) in dichloromethane. The reaction was kept at 0 °C for 2 h and monitored by thin-layer chromatography, and after the raw material was completely reacted, the resulting solution was processed to obtain 12 mg of product.

Synthesis of Example 17: Compound 5 (12 mg, 0.024 mmol) and Compound 6 (6 mg, 0.018 mmol), and p-toluenesulfonic acid (8 mg,0.042 mmol) were dissolved in 1 mL of N-methyl pyrrolidone and 2 mL of ethylene glycol monomethyl ether, and the reaction was warmed up to 100 °C under nitrogen atmosphere for 4 hours. The reaction was monitored by thin-layer chromatography, and after the raw material was completely reacted, the resulting solution was processed to obtain 3 mg of product. [M+H]⁺: 675.8. ¹H NMR (400 MHz, Chloroform-*d*) δ 10.42 (s, 0H), 9.97 (s, 0H), 8.91 (s, 0H), 8.70 (m, 1H), 7.68 (s, 1H), 7.33 (d, J = 8.2 Hz, 0H), 7.18 (m, 1H), 6.99 (m, 1H), 6.45 (m, 1H), 6.33 (m, 1H), 5.73 (m, 1H), 5.35 (m, 1H), 4.85 (m, 2H), 3.99 (s, 3H), 3.05 (m, 2H), 2.78 (m, 1H), 2.62(m,2H) , 2.35 (s, 3H), 2.22 (s, 3H), 2.10 - 1.95 (m, 2H).

### II. Biological tests and evaluation of compounds

### 1. Tests of compounds against cell proliferation

### 1.1 Cell proliferation inhibitory activity testing assays for EGFR and HER2 exon 20 insertion mutations:

The selected engineering cells Ba/F3-FL-EGFR-V769-D770 ins ASV, Ba/F3-FL-EGFR-D770-N771 ins SVD, Ba/F3-FL-EGFR-H773-V774 ins NPH, Ba/F3-FL-EGFR-A763-Y764 ins FQEA, Ba/F3-HER2-A775-G776 ins YVMA were provided by Hefei PreceDo Biomedical Technology Co., LTD and were validated prior to use in this study.

A 20× stock of the compound to be tested was prepared for use, with 9 concentrations in 3-fold gradient dilution, diluted from 1 µM.

The logarithmic growth phase cell suspension was inoculated into 96-well white cell culture plates with 95 µL volume per well (2000 cells/well). 5 µL of 20×Compound to be tested was added to the plate containing 95 µL of cell suspension according to the plate spreading diagram, and then mixed well. The plate was incubated in a 5% CO₂ incubator at 37 °C for 72 h. The proliferation inhibition activity of the compounds was determined by the CellTiter-Glo method, and the fluorescence value per well, RLU, was obtained from the SpectraMax Paradigm reading. Inhibition Rate data were processed using the following formula: Inhibition Rate (Inh%)=100-(RLU compound-RLU blank)/(RLU control-RLU blank)* 100%. The cell viability corresponding to different concentrations of compounds was calculated in EXCEL, and then GraphPad Prism software was used to plot the cell viability curve and calculate the IC₅₀ value. Table 5 shows the name and structure of the control compounds.

**Table 5 Name and structure of control compounds**

| Name | TAK-788 | DZD9008 |
|---|---|---|
| Structure | 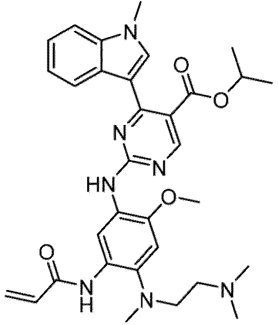 | 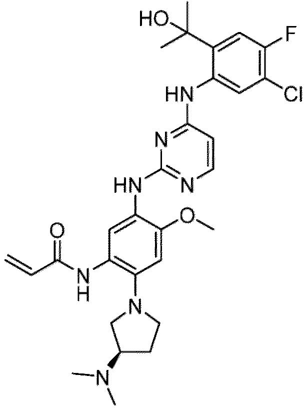 |

In the experimental results of Tables 6, 7 and 8, the IC₅₀ values were categorized into 3 groups based on the size of the IC₅₀ values, wherein A ≤ 30 nM, 30 nM < B ≤ 100 nM, and C > 100 nM.

Table 6 Activity test results of different compounds against proliferation of cells with EGFR exon 20 insertion mutations

| Compound | Ba/F3-FL-EGFR | | | |
|---|---|---|---|---|
| | V769-D770ins ASV | D770-N771ins SVD | H773-V774ins NPH | A763-Y764ins FQEA |
| DZD-9008 | A | B | B | A |
| TAK-788 | A | A | A | A |
| Example 1 | A | A | A | A |
| Example 2 | A | A | A | A |
| Example 3 | A | A | A | A |
| Example 5 | A | A | A | A |
| Example 7 | A | A | A | A |
| Example 8 | A | A | A | A |

**Table 7 inhibitory activity test results of different compounds against cells with HER2 exon 20 insertion mutation**

| Compound | DZD9008 | TAK-788 | Example 1 | Example 5 |
|---|---|---|---|---|
| Ba/F3-HER2-A775-G776 ins YVMA | B | A | A | A |

As can be seen from Table 6, the compounds in the Examples all showed excellent inhibition against EGFR exon 20 insertion mutations, superior to DZD9008 and comparable to the activity of TAK-788. As can be seen from Table 7, the cellular activity of Example 5 against HER2 exon 20 insertion mutations is comparable to that of TAK-788 and superior to that of DZD9008.

### 1.2 Cell proliferation inhibition activity test assay of compounds on cell lines NCI-H1975 and PC9:

The cell lines NCI-H1975 and PC9 selected for the experiments were provided by Sino-American Guanke Biotechnology (Beijing) Co. and were validated prior to use in this study.

A 10× stock of the compound to be tested was prepared for use, with 9 concentrations in 4-fold gradient dilution, diluted from 10 µM.

The logarithmic growth phase cell suspension was inoculated into a 96-well culture plate with 90 µL volume per well (2000 cells/well). 10 µL of 10×Compound to be tested was added to the plate according to the plate spreading diagram, and then mixed well. The plate was incubated in a 5% CO₂ incubator at 37 °C. The proliferation inhibitory activity of the compound was determined by the CellTiter-Glo method. Cell proliferation inhibition rate (Inhibition Rate) data were plotted using GraphPad Prism 8.0 software for cell viability curves and IC₅₀ values were calculated. The results were shown in Table 8:

**Table 8 Inhibitory activity test results of different compounds against cells with associated mutations**

| Compound | DZD9008 | TAK-788 | Example 1 | Example 5 |
|---|---|---|---|---|
| NCI-H1975 | A | A | A | A |
| PC9 | B | A | A | A |

As can be seen from Table 8, the compounds of the present invention have good inhibitory effect against cells of the cell lines NCI-H975 and PC9 and were superior to DZD9008.

### 2. In vivo pharmacodynamic experiments of compounds

### 2.1 Pharmacodynamic study on lung cancer LU0387PDX model

Lung cancer LU0387PDX model is a commonly used xenograft tumor model from Crown Bioscience, the experimental situation of this model is described as follows: after euthanasia of tumor-bearing mice, the tumor blocks were dissected out in aseptic condition, the blocks were cleaned to remove blood stains and connective tissues and necrotic parts, and the tumors were cut into small pieces of 2*2*2mm³, and inoculated with an adapter needle in sterilized experimental mice subcutaneously on the right dorsal side. Tumor growth was regularly observed, and when the tumors grew to an average volume of 100~200mm³, mice were randomly grouped according to tumor size and body weight for drug administration. Before the start of drug administration, all animals were weighed and the tumor volume was measured with vernier calipers. Mice were randomly grouped according to tumor volume to ensure that tumor volumes were similar between groups. Three mice in each group were administered orally once daily, and the inoculation site was observed after administration. Tumor volume and mouse body weight were measured twice a week.

The experimental design and results were shown in Table 9, wherein P.O refered to administered orally; QD: once a day; TGI (Tumor volume inhibition rate) = (1 - Tumor weight of the treatment group / Tumor weight of the control group)* 100%.

**Table 9 LU0387 model design and results**

| Group | | Dose of drug administration | Mode of administration | TGI (%) |
|---|---|---|---|---|
| 1 | vehicle control group | / | P.O, QD * 13 days | / |
| 2 | Example 1 | 30mg/kg | P.O, QD * 13 days | 79.01 |
| 3 | Example 1 | 50mg/kg | P.O, QD * 13 days | 97.44 |
| 4 | TAK-788 | 30mg/kg | P.O, QD * 13 days | 68.03 |
| 5 | Example 5 | 30mg/kg | P.O, QD * 13 days | 66.90 |
| 6 | DZD-9008 | 30mg/kg | P.O, QD * 13 days | 32.55 |

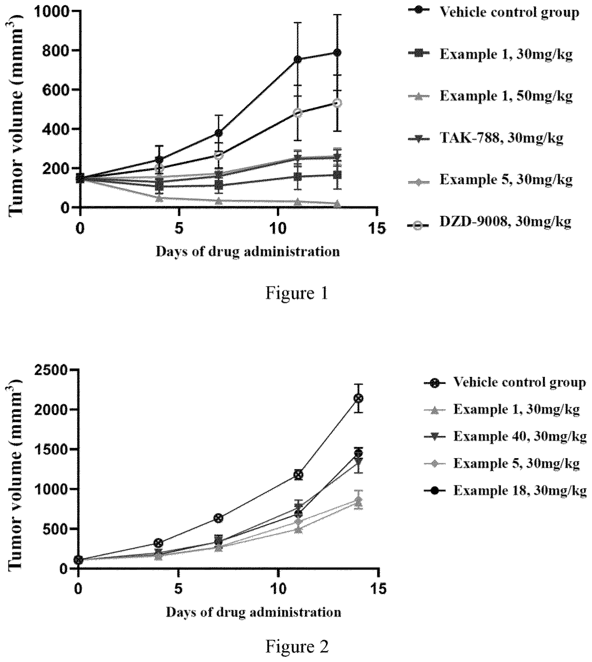

During the experiment, the tumor volume changed as days of administration went by, and the results were shown in Figure 1.

As can be seen in Figure 1, the efficacy of Example 1 was superior to that of TAK-788 and DZD9008 at the same dose in the LU0387 model. The efficacy of Example 5 was similar to that of TAK-788. As the dose of Example 1 increased, the efficacy was also significantly enhanced.

### 2.2 In vivo pharmacodynamic study of Ba/F3-EGFR-D770_N771 ins SVD engineered cell line in subcutaneous xenograft model

The cell line Ba/F3 EGFR D770_N771 ins SVD was cultured in 37°C, 5% CO₂ with RPMI1640 +10% fetal bovine serum+1% double antibody, and passaged 2-3 times a week. When the cell saturation was 80-90% and the number of cells met the requirement, the cells were collected. 0.2 mL(1 * 10⁶) cells were subcutaneously inoculated into the right back of 6-8 weeks old female nude mice weighing 18-22g. When the average tumor volume reached approximately 150-200 mm³, grouping and drug administration started. Before the start of drug administration, all animals were weighed and the tumor volume was measured with vernier calipers. Mice were randomly grouped according to tumor volume to ensure that tumor volumes were similar between groups. Three mice in each group were administered orally once daily, and the inoculation site was observed after administration. Tumor volume and mouse body weight were measured twice a week.

**Table 10 Grouping of Ba/F3 EGFR D770 N771 ins SVD models**

| Group | | Dose of drug administration | Mode of administration | TGI(%) |
|---|---|---|---|---|
| 1 | vehicle control group | / | P.O, QD * 14days | / |
| 2 | Example 1 | 30mg/kg | P.O, QD * 14days | 61.08 |
| 3 | Example 40 | 30mg/kg | P.O, QD * 14days | 37.84 |
| 4 | Example 5 | 30mg/kg | P.O, QD * 14days | 59.51 |
| 5 | Example 18 | 30mg/kg | P.O, QD * 14days | 32.22 |

Example 18 and Example 40 in the table were compounds having the structure of trans-3-membered-5-membered fused ring amine from application WO2021180238. The names and structures were shown in Table 11:

**Table 11 Names and structures of trans-structural compounds**

| Name | Example 40 | Example 18 |
|---|---|---|
| Structure | 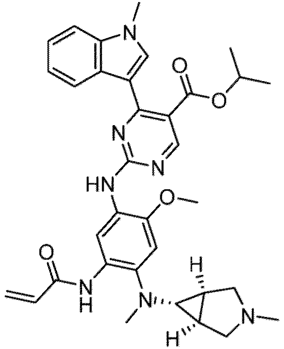 | 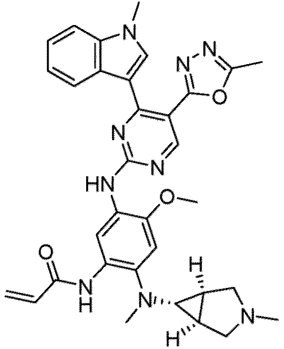 |

During the experiment, the tumor volume changed as the administration days went by, and the results were shown in Figure 2.

As can be seen in Figure 2, in the Ba/F3-EGFR-D770_N771 ins SVD cell transplantation model, the in vivo efficacy of both Example 1 and Example 5 at the same dose was much better than that of the compounds with trans-3-membered-5-membered fused ring amine structure in application WO2021180238, with the tumor inhibition rate increased by more than 1.6-fold.

In summary, the compounds in the present invention have good inhibitory effect on the exon 20 insertion mutation in EGFR or HER2, and also have good inhibitory effect on the cell proliferation of cell lines NCI-H1975 and PC9; and have good inhibitory effect on one or a combination of two or more of the exon 20 insertion mutation in EGFR, the exon 20 insertion mutation in HER2, the exon 19 deletion in EGFR, the exon 20 point mutation in EGFR, and the exon 21 point mutation in EGFR, and the drug thereof is expected to have a better therapeutic effect on related diseases.

### III. Structure confirmation and study on free base crystallization of Example 1.

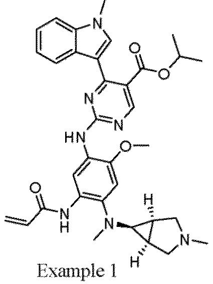

### 1. Structure confirmation

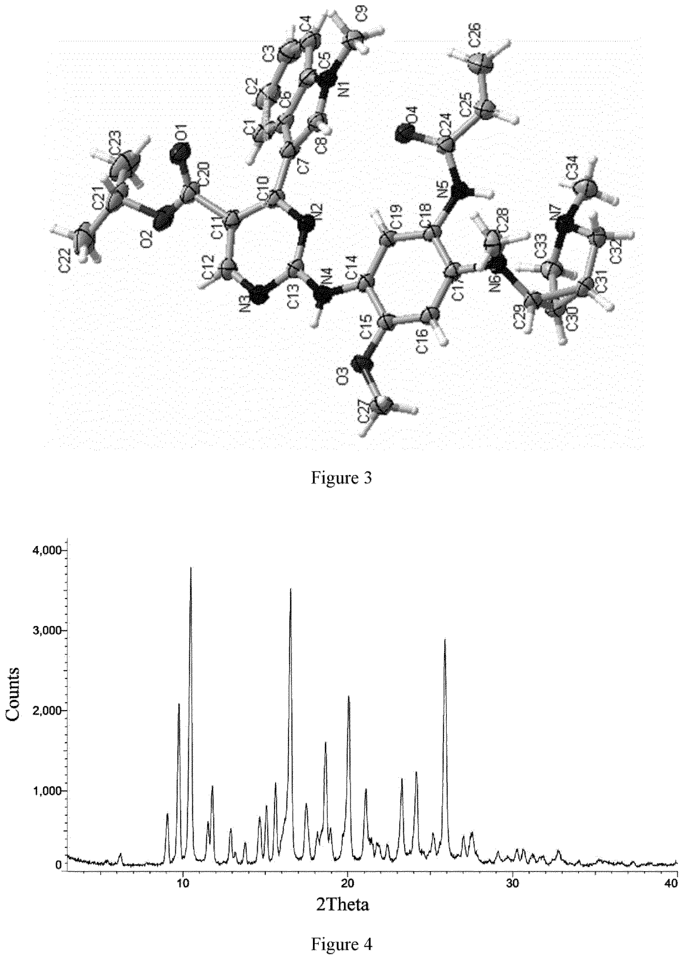

214 mg of the compound of Example 1 was added to 0.5 mL of dichloromethane and 0.5 mL of acetonitrile, dissolved to clear and filtered; the filtrate was placed into a sample vial with sealing, then the membrane was perforated and the vial was placed in a fume hood for slow evaporation to obtain granular crystals (free base crystalline form I). Single crystal diffraction was performed for structural confirmation, and the results were shown in Figure 3, which confirmed the compound conformation.

### 2. Study of the free base crystalline form of Example 1

The compound obtained in Example 1 was subjected to polycrystalline screening to search for its potential crystalline form, with a view to finding an excellent crystal form suitable for subsequent development. Taking the Example 1 obtained by synthesis as the starting material, the compound was detected and found to be a crystal with good crystallinity and an anhydrous crystalline form, which was named as free base crystalline form I. The amorphous free base compound was subsequently prepared by dry grinding. The above two types were used as starting materials to carry out crystalline form screening experiments.

### 2.1 Preparation and Characterization of Starting Materials.

### 2.1.1 Characterization of free base crystalline form I of the compound of Example 1

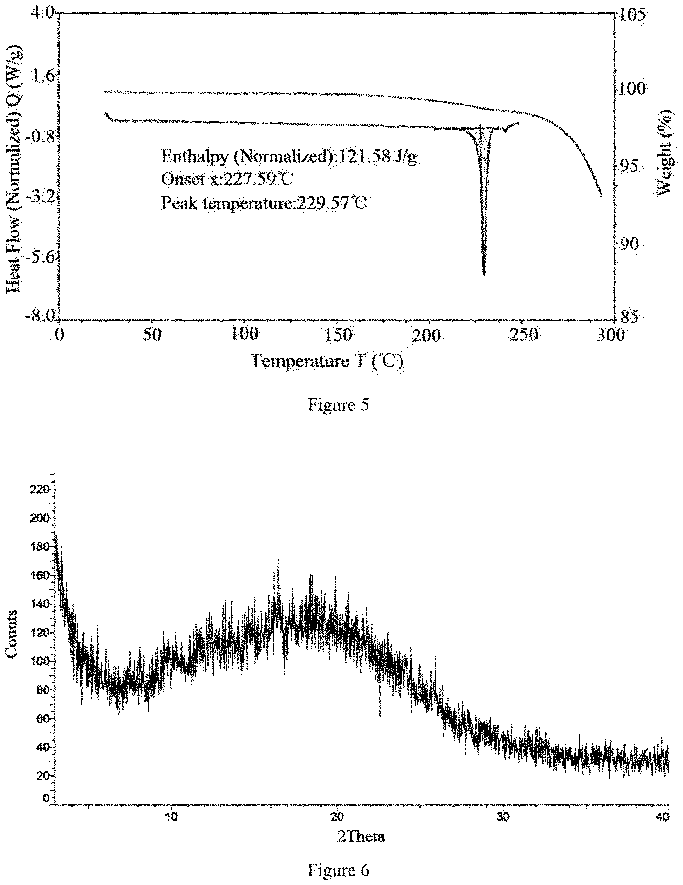

The starting material of the free base crystalline form I of the compound of Example 1 was fully characterized. The starting material was observed by polarized light microscopy (PLM) as irregularly shaped crystals with good crystallinity. Figure 4 shows the XRPD pattern of the free base crystalline form I, and Figure 5 shows the DSC and TGA overlay pattern of the crystalline form. The differential scanning calorimetry (DSC) curve has one endothermic peak at 230°C, which should be the melting peak. The thermogravimetric analysis (TGA) curve has no obvious weight loss before decomposition, which indicate that the crystalline form is an anhydrous crystalline form.

**Table 12 X-ray powder diffraction peak data of the free base crystalline form I**

| 2θ(°) ± 0.2 | Relative Intensity (100%) | 2θ(°) ± 0.2 | Relative Intensity (100%) | 2θ(°) ± 0.2 | Relative Intensity (100%) |
|---|---|---|---|---|---|
| 6.195 | 3.2 | 18.163 | 9.2 | 27.562 | 9.7 |
| 9.071 | 16.3 | 18.660 | 40.5 | 28.603 | 1.3 |
| 9.765 | 52.8 | 18.964 | 11.2 | 29.112 | 4.0 |
| 10.488 | 100.0 | 20.067 | 54.3 | 29.693 | 2.4 |
| 11.530 | 13.4 | 21.103 | 23.6 | 30.264 | 4.1 |
| 11.795 | 25.8 | 21.399 | 6.4 | 30.659 | 4.3 |
| 12.912 | 11.6 | 21.833 | 5.2 | 31.192 | 3.2 |
| 13.181 | 3.6 | 22.397 | 5.4 | 31.703 | 1.7 |
| 13.788 | 7.2 | 23.290 | 28.3 | 32.799 | 4.1 |
| 14.667 | 15.6 | 24.164 | 30.4 | 34.024 | 1.3 |
| 15.076 | 18.9 | 24.512 | 2.5 | 35.238 | 2.2 |
| 15.629 | 26.0 | 25.171 | 8.9 | 37.325 | 1.6 |
| 16.537 | 91.8 | 25.897 | 73.2 | 38.347 | 0.8 |
| 17.495 | 18.1 | 27.032 | 7.6 | | |

### 2.1.2 Solubility test of free base crystalline form I of Example 1

The solubility of the free base crystalline form I of the compound of Example 1 was crudely determined by visual inspection in 18 solvents at room temperature. The results were shown in Table 13. The free base crystalline form I was only highly soluble in dichloroethane and dichloromethane and poorly soluble in most of the solvents tested.

**Table 13 Visual inspection results of the solubility of the free base crystalline form I of Example 1**

| Number | Solvent | Solubility mg/mL | Number | Solvent | Solubility mg/mL |
|---|---|---|---|---|---|
| 1 | Dichloromethane | >107 | 10 | 1,4-dioxane | <0.27 |
| 2 | Dichloroethane | 61.75-123.50 | 11 | Tert-butyl methyl ether | <0.33 |
| 3 | N-Methyl pyrrolidone | 18.25-27.38 | 12 | Ethyl acetate | <0.29 |
| 4 | Acetone | 2.22-4.44 | 13 | Methanol | <0.40 |
| 5 | Dimethylsulfoxide | 2.60-2.93 | 14 | Isopropyl acetate | <0.30 |
| 6 | Butanone | 2.78-4.44 | 15 | Cyclohexane | <0.33 |
| 7 | Tetrahydrofuran | 2.55-3.19 | 16 | Water | <0.27 |
| 8 | Acetonitrile | 0.93-1.01 | 17 | Isopropanol | <0.35 |
| 9 | Ethanol | <0.30 | 18 | n-Heptane | <0.30 |

### 2.1.3 Preparation and characterization of amorphous raw material

736 mg of the compound of Example 1 (free base crystalline form I) was weighed and dry-grinded for 5 hours to successfully afford 669.86 mg of amorphous product. XRPD detection was performed and the results are shown in Figure 6.

### 2.2. Usage and parameters of physicochemical detection instruments applied in characterization

### 2.2.1 X-ray powder diffraction (XRPD)

XRPD diffraction pattern were acquired by a Bruker D2 Phaser type. The sample to be measured was placed on a smooth, background-free silicon substrate for sampling. The measurement parameters are shown in Table 14.

**Table 14 XRPD method parameters**

| Instruments | Bruker, D2 Phaser | Tube voltage/current | 30 KV/10 mA |
|---|---|---|---|
| Emission wavelength | Cu K α (=1.5418 Å) | Main optical path axial sollar slit | 2.5° |
| Sensing mode | Step measurement | Secondary optical path axial sollar slit | 2.5° |
| Scanning axis | θs- θd | Detector slit | 5.827° |
| Scanning angle | 3-40° (2θ) | Divergent slit width | 0.6 mm |
| Scanning step | 0.02° (2θ) | Anti-Scattering Slit | 0 mm |
| Scanning speed | 0.2 s/step | Rotation | Open |

### 2.2.2 Polarized light microscopy (PLM)

PLM analysis was carried out using an Optec optical microscope BK-Pol. A small amount of sample was taken and placed on a slide, dispersed with a drop of silicone oil, then covered with a glass slide and observed under the microscope.

### 2.2.3 Differential scanning calorimetric analysis (DSC)

The DSC curves were acquired by the DSC 250 model of TA Instrument. The test method of DSC 250 model was as follows: an appropriate amount of sample was weighed precisely into a perforated aluminum crucible, the temperature was increased from 25 °C to the final temperature of 300 °C at an increase rate of 10 °C/min, and purged with nitrogen gas at a flow rate of 50 mL/min.

### 2.2.4 Thermogravimetric analysis (TGA)

TGA data were acquired by the TGA 550 model of TA Instrument. An appropriate amount of sample was taken into the aluminum crucible which was peeled in advance, and the temperature increased from room temperature to 300 °C at an increase rate of 10 °C/min, the balance chamber was purged with nitrogen at a flow rate of 40 mL/min, and the sample chamber was purged with nitrogen at a flow rate of 25 mL/min.

### 2.3 Study of free base polycrystalline forms of the compound of Example 1.

It was mainly prepared by suspension crystal transformation, anti-solvent precipitation, high and low temperature cycling and evaporating crystallization.

Suspension crystal transformation was carried out according to the visual solubility results of the compound of Example 1, and the free base crystalline form I and the amorphous crystalline form were used as starting materials at 25 °C and 50 °C, respectively, and the preparation of crystalline forms was performed in a selected single solvent (solvent selected from dichloromethane, 1,4-dioxane, dichloroethane, tert-butyl methyl ether, N-methylpyrrolidinone, ethyl acetate, acetone, methanol, dimethylsulfoxide, isopropyl acetate, butanone , cyclohexane, tetrahydrofuran, water, acetonitrile, isopropanol, ethanol, n-heptane) or a mixed solvent. A total of two crystalline forms were obtained, i.e., free base crystalline form I and free base crystalline form II.

Anti-solvent precipitation was carried out based on the visual solubility results of the compound of Example 1 by using dichloromethane and dichloroethane as good solvents, and then adding different anti-solvents to each of them under stirring at room temperature (~25 °C) to prepare the crystalline forms. A total of two crystalline forms were obtained, i.e., free base crystalline form I and free base crystalline form III.

High and low temperature cycling was performed by adding a solvent (solvents selected from dichloromethane, 1,4-dioxane, dichloroethane, tert-butyl methyl ether, N-methylpyrrolidone, ethyl acetate, acetone, methanol, dimethyl sulfoxide, isopropyl acetate, butanone, cyclohexane, tetrahydrofuran, water, acetonitrile, isopropanol, ethanol, and n-heptane) to the compound, and then stirred under temperature cycling of 50 °C~5 °C to prepare crystalline forms. A total of two crystalline forms were obtained, i.e., free base crystalline form I and free base crystalline form II.

Evaporating crystallization was carried out by adding a solvent to the compound, the obtained solution was filtered, and the filtrate was added dropwise to a sample vial, which was sealed with sealing membrane and placed in a fume hood for slow evaporation after the sealing membrane covered on the mouth of the vial was perforated. A total of two crystalline forms were obtained, i.e., free base crystalline form I and free base crystalline form IV The free base crystalline form IV was heated to 150 °C to desolventize and a new crystalline form, named free base crystalline form V, was prepared.

### 2.3.1 Preparation and characterization of free base crystalline form I

Free base crystalline form I can be obtained in most solvents, and the compound obtained in Example 1 are free base crystalline form I. The characterization results are shown in Figures 4 and 5.

### 2.3.2 Preparation and characterization of free base crystalline form II

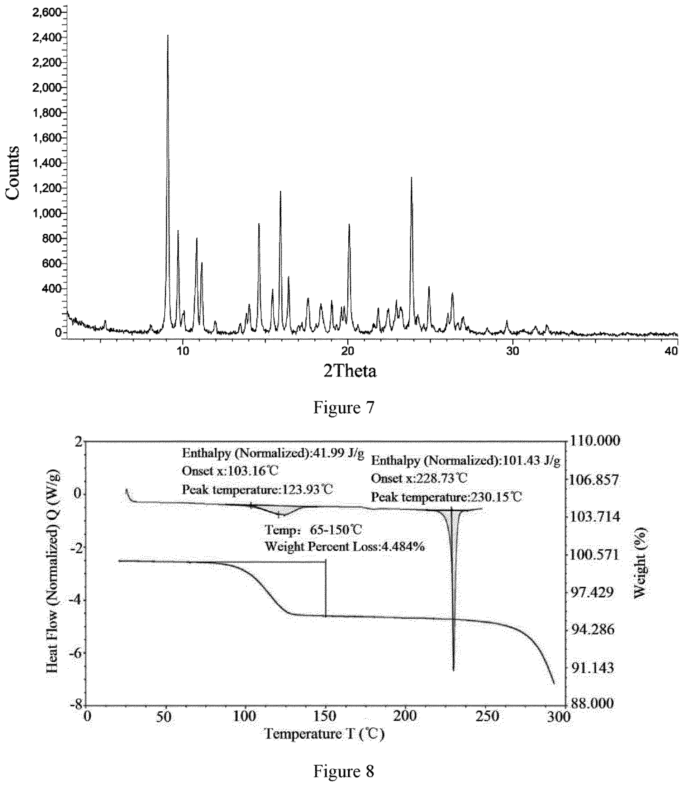

Free base crystalline form II can be obtained in certain solvent systems containing ethanol, using amorphous compound as raw material. Free base crystalline form II can be obtained by suspension crystal transformation in a single solvent ethanol. The characterization results are shown in Figure 7 and Figure 8, the DSC curve of free base crystalline form II has two endothermic peaks at 124 °C and 230 °C, the TGA curve has a weight loss of 4.48% at from 65 to 150 °C, and the free base crystalline form II was obtained in the solvent system of ethanol. It was inferred that the free base crystalline form II should be an ethanol solvate. The endothermic peak at 124 °C on the DSC curve should be the desolvation peak.

**Table 15 X-ray powder diffraction data of free base crystalline form II**

| 2θ(°) ± 0.2 | Relative Intensity (100%) | 2θ(°) ± 0.2 | Relative Intensity (100%) | 2θ(°) ± 0.2 | Relative Intensity (100%) |
|---|---|---|---|---|---|
| 5.298 | 2.5 | 17.215 | 3.2 | 24.233 | 5.8 |
| 8.075 | 1.8 | 17.593 | 11.1 | 24.596 | 1.7 |
| 9.092 | 100.0 | 18.077 | 2.7 | 24.918 | 16.7 |
| 9.720 | 36.1 | 18.367 | 9.0 | 25.162 | 2.3 |
| 10.036 | 5.4 | 19.031 | 10.8 | 26.067 | 6.8 |
| 10.843 | 31.1 | 19.313 | 2.4 | 26.337 | 14.7 |
| 11.151 | 22.9 | 19.608 | 8.3 | 26.658 | 2.7 |
| 11.975 | 3.6 | 19.778 | 9.1 | 27.011 | 5.1 |
| 13.483 | 2.4 | 20.083 | 37.7 | 27.268 | 1.6 |
| 13.853 | 6.7 | 20.614 | 2.5 | 28.449 | 2.2 |
| 14.036 | 9.1 | 21.573 | 2.8 | 29.643 | 5.0 |
| 14.621 | 37.5 | 21.839 | 8.8 | 31.371 | 2.0 |
| 15.440 | 14.1 | 22.443 | 6.9 | 32.074 | 2.9 |
| 15.922 | 49.1 | 22.943 | 10.8 | 33.583 | 1.0 |
| 16.417 | 20.2 | 23.213 | 7.6 | 36.957 | 1.1 |
| 17.018 | 1.9 | 23.865 | 54.8 | | |

### 2.3.3 Preparation and Characterization of Free Base Crystalline Forms III

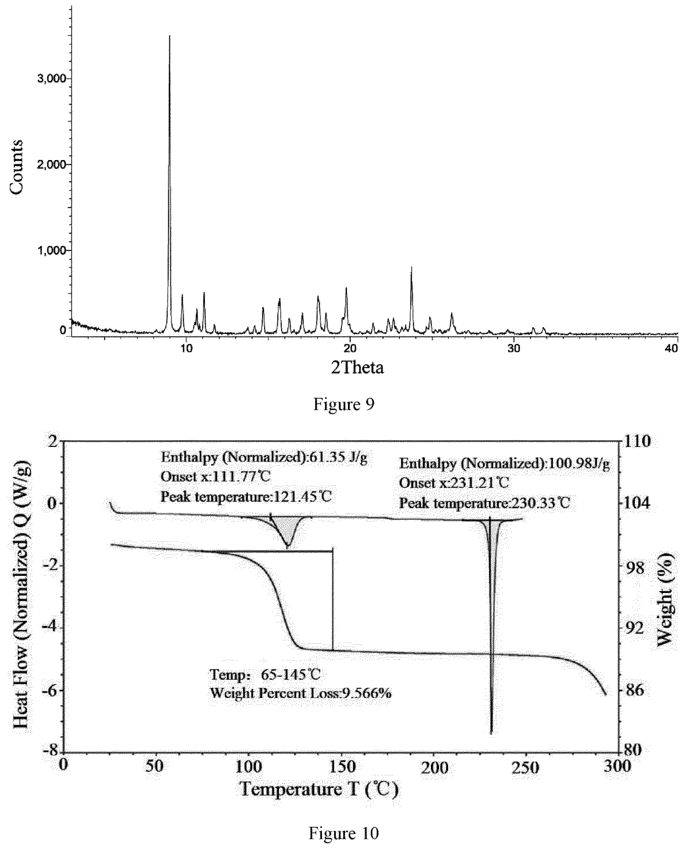

Free base crystalline form III can be obtained in certain solvent systems containing isopropanol, and in the preparation of crystalline form by anti-solvent precipitation, free base crystalline form III was found in dichloromethane/isopropanol or dichloroethane/isopropanol systems. The characterization results are shown in Figure 9 and Figure 10. The DSC curve of free base crystalline form III has two endothermic peaks at 121 °C and 230 °C, the TGA curve has a weight loss of 9.57% at from 75-145 °C, and the free base crystalline form III was transformed to free base crystalline form I after desolvation by heating to 150 °C with XRPD. The free base crystalline form III was obtained in a solvent system of isopropanol. In summary, it was inferred that the free base crystalline form III should be an isopropanol solvate, and the endothermic peak at 121 °C on the DSC curve should be the desolvation peak.

**Table 16 X-ray powder diffraction data of free base crystalline form III**

| 2θ(°) ± 0.2 | Relative Intensity (100%) | 2θ(°) ± 0.2 | Relative Intensity (100%) | 2θ(°) ± 0.2 | Relative Intensity (100%) |
|---|---|---|---|---|---|
| 8.169 | 1.1 | 17.487 | 1.1 | 24.678 | 2.1 |
| 8.972 | 100.0 | 18.041 | 12.1 | 24.880 | 5.8 |
| 9.758 | 12.6 | 18.526 | 7.0 | 25.204 | 1.4 |
| 10.576 | 6.6 | 19.596 | 4.2 | 25.973 | 1.0 |
| 10.793 | 3.2 | 19.769 | 16.7 | 26.204 | 7.3 |
| 11.088 | 13.6 | 19.934 | 2.9 | 26.978 | 1.0 |
| 11.713 | 2.9 | 20.602 | 0.5 | 27.210 | 1.0 |
| 13.736 | 1.8 | 21.403 | 3.3 | 27.941 | 0.7 |
| 14.157 | 2.3 | 21.732 | 0.8 | 28.464 | 1.1 |
| 14.689 | 9.0 | 22.331 | 4.6 | 29.615 | 1.3 |
| 15.701 | 12.2 | 22.712 | 4.1 | 31.181 | 2.0 |
| 16.281 | 5.2 | 23.155 | 1.9 | 31.794 | 2.7 |
| 16.565 | 1.1 | 23.377 | 3.0 | 32.597 | 0.7 |
| 17.080 | 7.0 | 23.741 | 26.6 | | |

### 2.3.4 Preparation and Characterization of Free Base Crystalline Form IV

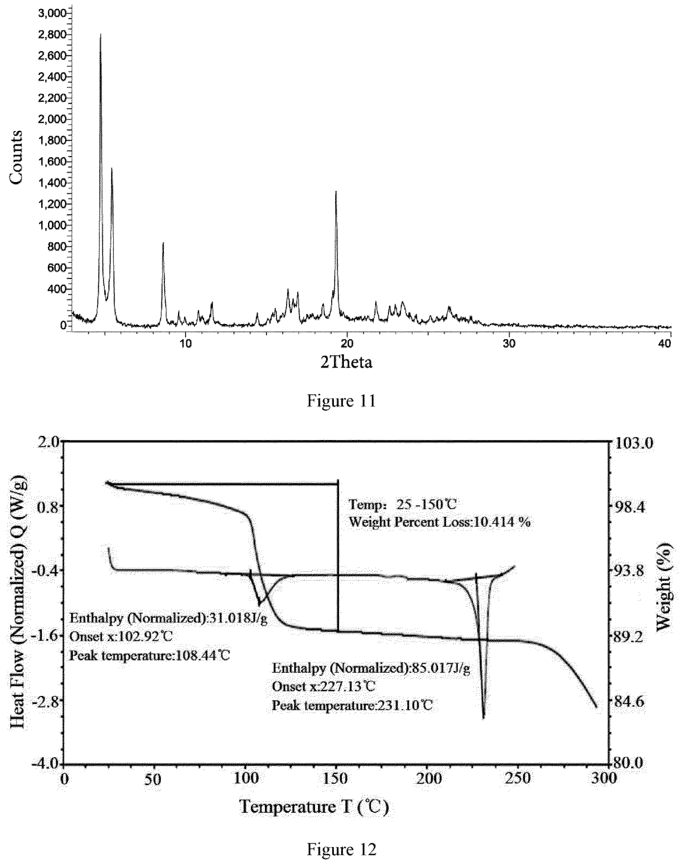

Free base crystalline form IV can be obtained in certain solvent systems containing dichloroethane. 92.28 mg of raw material was weighed and added to a sample vial, 5.0 mL of dichloroethane was added to dissolve it, the top of the sample vial was covered with a sealing membrane, perforated and then placed in a fume hood for slow evaporation, thus the crystalline form IV was prepared by evaporating crystallization. The characterization results are shown in Figure 11 and Figure 12, the DSC curve of free base crystalline form IV has two endothermic peaks at 108 °C and 231 °C, and the TGA curve has a weight loss of 10.41% at from 25-150 °C. The ¹H NMR result shows only 6.14% of dichloroethane solvent residue, from which it can be inferred that the sample might contain 4.27% of water. In summary, it is inferred that the free base crystalline form IV may be a solvate of mixed dichloroethane and water.

**Table 17 X-ray powder diffraction data of free base crystalline form IV**

| 2θ(°) ± 0.2 | Relative Intensity (100%) | 2θ(°) ± 0.2 | Relative Intensity (100%) | 2θ(°) ± 0.2 | Relative Intensity (100%) |
|---|---|---|---|---|---|
| 4.761 | 100.0 | 15.402 | 5.1 | 19.759 | 3.0 |
| 5.460 | 55.1 | 15.567 | 4.8 | 21.310 | 1.2 |
| 8.619 | 31.7 | 16.029 | 3.3 | 21.781 | 6.9 |
| 9.174 | 2.0 | 16.337 | 14.1 | 22.612 | 6.1 |
| 9.595 | 5.7 | 16.659 | 9.6 | 22.984 | 6.0 |
| 9.962 | 3.0 | 16.938 | 11.7 | 23.420 | 7.4 |
| 10.804 | 5.4 | 17.540 | 1.8 | 23.839 | 1.3 |
| 11.012 | 1.9 | 17.845 | 2.5 | 25.136 | 2.9 |
| 11.618 | 5.4 | 18.502 | 4.9 | 25.511 | 2.5 |
| 14.435 | 5.3 | 19.109 | 11.7 | 26.272 | 6.6 |
| 15.099 | 2.1 | 19.311 | 53.9 | | |

### 2.3.5 Preparation and characterization of free base crystalline form V

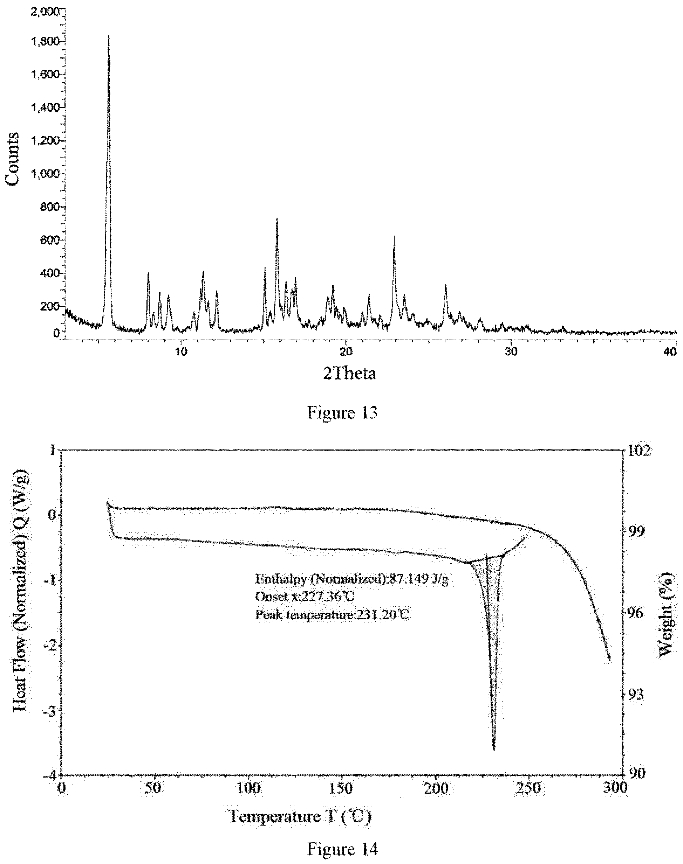

The free base crystalline form IV was characterized by XRPD after desolvation by heating to 150 °C, and showed that it was transformed into a new crystalline form, named free base crystalline form V The characterization results are shown in Figure 13 and Figure 14, the DSC curve of free base crystalline form V has an endothermic peak at 231°C, and the TGA curve has no obvious weight loss before decomposition. In summary, it is inferred that the free base crystalline form V should be an anhydrous crystalline form.

**Table 18 X-ray powder diffraction data of free base crystalline form V**

| 2θ(°) ± 0.2 | Relative Intensity (100%) | 2θ(°) ± 0.2 | Relative Intensity (100%) | 2θ(°) ± 0.2 | Relative Intensity (100%) |
|---|---|---|---|---|---|
| 5.622 | 100.0 | 16.363 | 14.9 | 22.903 | 33.3 |
| 8.021 | 20.5 | 16.726 | 13.1 | 23.526 | 12.3 |
| 8.328 | 5.9 | 16.935 | 17.0 | 24.074 | 3.5 |
| 8.706 | 14.1 | 17.756 | 2.6 | 25.048 | 2.5 |
| 9.243 | 12.9 | 18.486 | 3.7 | 26.027 | 17.5 |
| 9.743 | 1.5 | 18.907 | 10.8 | 26.335 | 5.8 |
| 10.776 | 6.9 | 19.214 | 14.4 | 26.877 | 6.4 |
| 11.341 | 21.2 | 19.630 | 4.7 | 27.111 | 4.5 |
| 11.638 | 7.9 | 19.924 | 4.1 | 27.481 | 1.6 |
| 12.159 | 13.0 | 20.980 | 5.7 | 28.113 | 2.7 |
| 15.082 | 24.3 | 21.382 | 11.5 | 29.449 | 3.1 |
| 15.409 | 6.4 | 21.671 | 1.9 | 30.927 | 2.9 |
| 15.815 | 41.6 | 22.054 | 4.4 | 33.106 | 3.4 |

### 3. Crystallographic evaluation of free base crystalline form I

In the screening process, only two kind of anhydrous crystalline forms were found, i.e., free base crystalline form I and free base crystalline form V Two solvents, acetone and acetonitrile was used to prepare saturated solutions, and then at 25 °C or 50 °C, the two were subjected to competitive pulping, it can be inferred from the results that the free base crystalline form I may be the thermodynamically stable crystalline form of the compound. And the free base crystalline form V was not obtained directly in the screening experiments, but only obtained from the desolvation of free base crystalline form IV Therefore, only free base crystalline form I was subjected to crystallographic evaluation, including dry grinding, wet grinding, tablet pressing (30 MPa), stability and hygroscopicity studies.

It was found by the grinding experiment that the free base crystalline form I transformed into amorphous state after 5 min of dry grinding; no significant change in crystallinity was observed after 5 min of wet grinding with water; and a slight decrease in crystallinity was observed after 5 min of wet grinding with ethanol.

In the tablet pressing experiment, when the pressure was 30 MPa, the crystallinity slightly decreased.

In the stability experiment, when the free base crystalline form I was left open at 80 °C for 3 days, 60 °C for 7 days, 25 °C/60 %RH for 7 days, 40 °C/75 %RH for 7 days, and 25 °C/90 ± 5 %RH for 7 days, the samples were taken for XRPD detection, respectively. It was found that there was no significant change in the free base crystalline form I, and there was no change in the results of the liquid chromatography thereof.

Dynamic water vapor adsorption (DVS) test was performed on the free base crystalline form I. The results showed that the free base crystalline form I was slightly hygroscopic with a weight gain by water absorption of 0.66% at 80 % RH.

In summary, the spatial configuration of such compound of Formula 1 was determined, and in particular the spatial structure of Example 1 was precisely determined. In the crystallographic screening studies performed on the compound of Example 1, a total of two anhydrous crystalline forms (free base crystalline form I and free base crystalline form V) and three solvates (free base crystalline form II, free base crystalline form III and free base crystalline form IV) were found. In the experiment, it was found that free base crystalline form I is a more stable anhydrous crystalline form with stable solid state properties and slight hygroscopicity, which can be used for subsequent drug development.

## Claims

1. A compound for use as kinase inhibitor, wherein the compound for use as kinase inhibitor is a compound of formula 1, or a deuterated compound thereof, or a pharmaceutically acceptable salt, a solvate or a prodrug thereof: 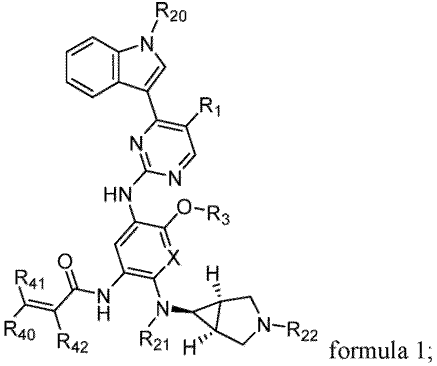 in formula 1, X is selected from CH and N;
R₁ is selected from 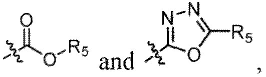 and R₅ is H, C1-C3alkyl, or C1-C3fluoroalkyl;
R₂₀, R₂₁, and R₂₂ are each independently selected from methyl and deuteromethyl;
R₃ is selected from C1-C3alkyl and C1-C3haloalkyl; and
R₄₀, R₄₁, and R₄₂ are each independently selected from H, D and F.

2. The compound for use as kinase inhibitor according to claim 1, wherein the compound is a compound of formula 2, or a deuterated compound thereof, or a pharmaceutically acceptable salt, a solvate or a prodrug thereof:

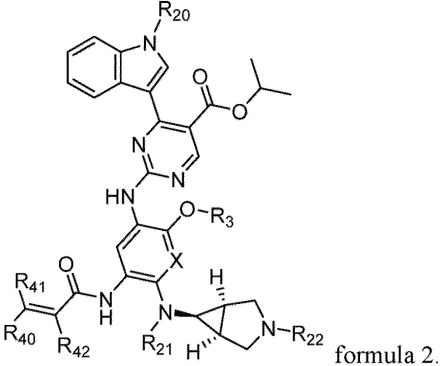

3. The compound for use as kinase inhibitor according to claim 2, wherein in formula 2, X is selected from CH and N; R₃ is selected from -CH₃, -CH₂CH₃ and -CH₂CF₃; R₄₀ and R₄₁ are H, and R₄₂ is selected from Hand F.

4. The compound for use as kinase inhibitor according to claim 1, wherein the compound is a compound of formula 3, or a deuterated compound thereof, or a pharmaceutically acceptable salt, a solvate or a prodrug thereof:

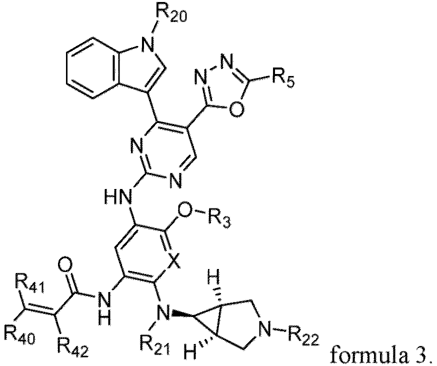

5. The compound for use as kinase inhibitor according to claim 4, wherein in formula 3, X is selected from CH and N; R₃ is -CH₃, -CH₂CH₃ or -CH₂CF₃; R₄₀ and R₄₁ are H, R₄₂ is selected from Hand F, and R₅ is selected from -CH₃ and -CF₃.

6. The compound for use as kinase inhibitor according to claim 1, wherein the compound of formula 1 is: 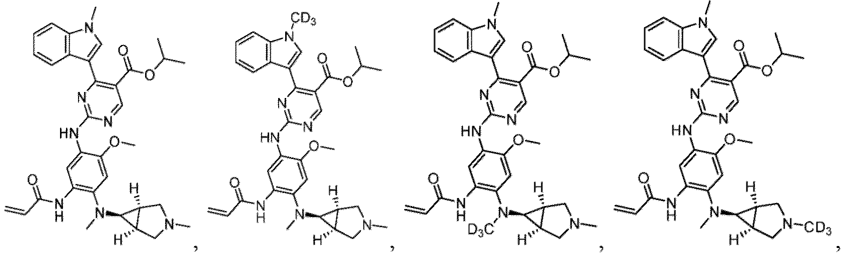 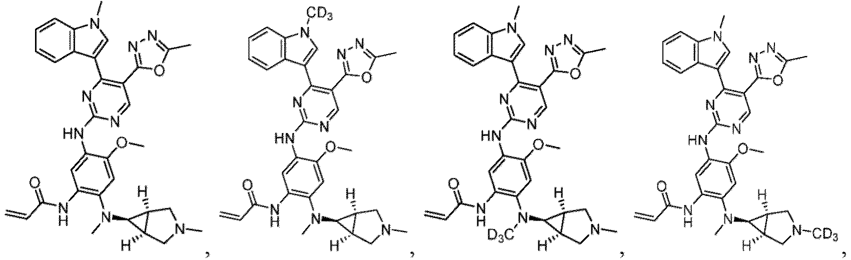 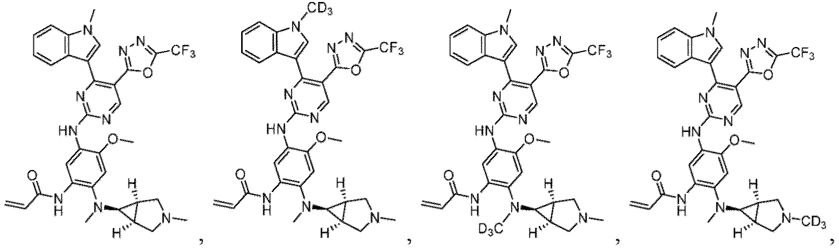 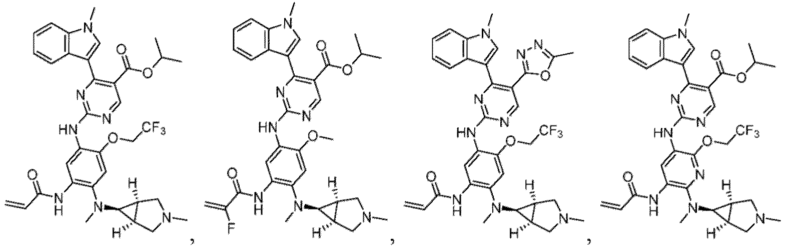 or

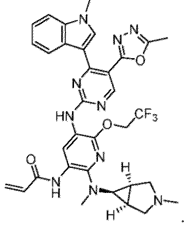

7. The compound for use as kinase inhibitor according to any of claims 1-6, wherein the compound is a crystalline, an amorphous or a solvate; the solvent contained in the solvate is a non-aqueous solvent or a mixture of non-aqueous solvent and water.

8. The compound for use as kinase inhibitor according to claim 7, wherein the compound has a structure of formula A: 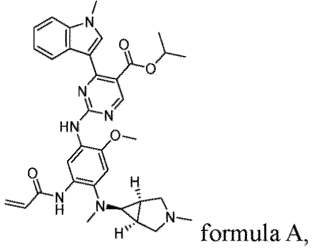 the crystalline form is free base crystalline form I, X-ray powder diffraction pattern of the crystalline form has characteristic diffraction peaks at 2θ of 9.76° ± 0.2°, 10.45° ± 0.2°, 16.54° ± 0.2°, 18.66° ± 0.2°, 20.07° ± 0.2°, and 25.90° ± 0.2°.

9. The compound for use as kinase inhibitor according to claim 8, wherein the X-ray powder diffraction pattern of the free base crystalline form I has characteristic diffraction peaks at 2θ of 9.07° ± 0.2°, 9.76° ± 0.2°, 10.45° ± 0.2°, 11.53° ± 0.2°, 11.80° ± 0.2°, 12.91° ± 0.2°, 13.79° ± 0.2°, 14.67° ± 0.2°, 15.08° ± 0.2°, 15.63° ± 0.2°, 16.54° ± 0.2°, 17.50° ± 0.2°, 18.66° ± 0.2°, 20.07° ± 0.2°, 21.10° ± 0.2°, 23.29° ± 0.2°, 24.16° ± 0.2°, and 25.90° ± 0.2°.

10. A use of the compound used as kinase inhibitor according to any of claims 1-9 in the preparation of a medication for the treatment of related diseases caused by EGFR mutations and/or HER2 mutations.

11. The use according to claim 10, wherein the EGFR mutation and/or HER2 mutation comprises one or a combination of two or more of a exon 20 insertion mutation in EGFR, an exon 20 insertion mutation of HER2, a exon 19 deletion in EGFR, a exon 21 point mutation in EGFR, and a exon 20 point mutation in EGFR.

12. The use according to claim 11, wherein the EGFR mutation and/or HER2 mutation is selected from EGFR Del 19/T790M/C797S mutation, EGFR L858R/T790M/C797S mutation.

13. The use according to claim 10, 11 or 12, wherein the disease is a cancer caused by said EGFR mutation and/or HER2 mutation.
